# EUROPEAN PATENT SPECIFICATION

(11) **EP 3 922 251 B1**
(45) Date of publication and mention of the grant of the patent: **28.05.2025**
(21) Application number: 20798403.0
(22) Date of filing: 23.04.2020
(51) Int. Cl.: A61K 31/58, A61K 31/56, A61K 31/704, A61K 45/06, A61P 35/00, A61K 9/00, A61K 9/06, A61K 39/39, A61K 39/395

(54) **CANCER IMMUNOTHERAPHY ADJUVANT**
ADJUVANS FÜR KREBSIMMUNOTHERAPIE
ADJUVANT D'IMMUNOTHÉRAPHIE ANTICANCÉREUSE

(30) Priority: 29.04.2019 KR 20190049990
(43) Date of publication of application: 15.12.2021
(73) Proprietor: Industry-Academic Cooperation Foundation, Yonsei University, Seoul 03722 (KR); CURACLE Co., Ltd., Seoul 06694 (KR)
(72) Inventor: KWON, Young-Guen, Seoul 04400 (KR); ZHANG, Haiying, Seongnam-si Gyeonggi-do 13449 (KR); PARK, Songyi, Seoul 02144 (KR); NOH, Minyoung, Seoul 01336 (KR); KIM, Yeomyeong, Seongnam-si Gyeonggi-do 13449 (KR); KIM, Myung-Hwa, Seongnam-si Gyeonggi-do 13449 (KR); AHN, Koo Hyeon, Seongnam-si Gyeonggi-do 13449 (KR); PYO, Jung-In, Seongnam-si Gyeonggi-do 13449 (KR)
(74) Representative: EP&C
(86) International application number: PCT/KR2020/005368
(87) International publication number: WO 2020/222461

(56) References cited:
- JP-A- H07 165 582
- LEE KEUNHO ET AL: "Combined effect of vascular-leakage-blocker Sac-1004 and antiangiogenic drug sunitinib on tumor angiogenesis", BIOCHEMICAL AND BIOPHYSICAL RESEARCH COMMUNICATIONS, ELSEVIER, AMSTERDAM NL, vol. 450, no. 4, 5 July 2014 (2014-07-05), pages 1320 - 1326, XP029044903, ISSN: 0006-291X, DOI: 10.1016/J.BBRC.2014.06.139
- XIN HONG ET AL: "Sunitinib Inhibition of Stat3 Induces Renal Cell Carcinoma Tumor Cell Apoptosis and Reduces Immunosuppressive Cells", CANCER RESEARCH, vol. 69, no. 6, 15 March 2009 (2009-03-15), US, pages 2506 - 2513, XP055908022, ISSN: 0008-5472, Retrieved from the Internet <URL:https://aacrjournals.org/cancerres/article/69/6/2506/552946/Sunitinib-Inhibition-of-Stat3-Induces-Renal-Cell> DOI: 10.1158/0008-5472.CAN-08-4323
- PARK SONGYI ET AL: "CU06-1004-Induced Vascular Normalization Improves Immunotherapy by Modulating Tumor Microenvironment via Cytotoxic T Cells", FRONTIERS IN IMMUNOLOGY, vol. 11, 26 January 2021 (2021-01-26), XP055908016, Retrieved from the Internet <URL:https://www.ncbi.nlm.nih.gov/pmc/articles/PMC7874050/pdf/fimmu-11-620166.pdf> DOI: 10.3389/fimmu.2020.620166
- AGRAWAL, V. ET AL.: "Direct endothelial junction restoration results in significant tumor vascular normalization and metastasis inhibition in mice", ONCOTARGET, vol. 5, no. 9, 2014, pages 2761 - 2777, XP055749305, DOI: 10.18632/oncotarget.1942
- MAHARJAN, S. ET AL.: "Sac-1004, a novel vascular leakage blocker, enhances endothelial barrier through the cAMP/Rac/cortactin pathway", BIOCHEMICAL AND BIOPHYSICAL RESEARCH COMMUNICATIONS, vol. 435, 2013, pages 420 - 427, XP028594795, DOI: 10.1016/j.bbrc.2013.04.104
- ZHANG, H. ET AL.: "Sac-1004, a vascular leakage blocker, reduces cerebral ischemia-reperfusion injury by suppressing blood-brain barrier disruption and inflammation", JOURNAL OF NEUROINFLAMMATION, vol. 14, no. 122, 2017, pages 1 - 15, XP055749351, DOI: 10.1186/s12974-017-0897-3
- LEE, K. ET AL.: "Combined effect of vascular-leakage-blocker Sac-1004 and antiangiogenic drug sunitinib on tumor angiogenesis", BIOCHEMICAL AND BIOPHYSICAL RESEARCH COMMUNICATIONS, vol. 450, 2014, pages 1320 - 1326, XP029044903, DOI: 10.1016/j.bbrc.2014.06.139

## Description

### BACKGROUND OF THE INVENTION

### 1. Field of the Invention

The present invention relates to a cancer immunotherapy adjuvant.

### 2. Description of the Related Art

Cancer is a disease characterized by abnormal, localized cell growth that has the potential to spread throughout the body. There are many types of cancer including lung cancer, bladder cancer, prostate cancer, pancreatic cancer, cervical cancer, brain cancer, stomach cancer, colorectal cancer and melanoma. In the past, the most common methods to treat oncological cancer (oncology) were surgery, radiation therapy or chemotherapy. However, it has recently been demonstrated that cancer immunotherapy has many promises as a treatment for oncology.

Cancer immunotherapy is a branch of oncology in which the immune system is used to treat cancer, as opposed to conventional treatment methods in which tumors are directly excised or treated. This therapeutic concept is based on the identification of many proteins on the surface of T cells that act to inhibit the immune function of these cells.

The most fundamental problem in tumor immunity is how to activate the immune system to recognize and eliminate antigens. In this respect, a novel method of genetically engineering tumor cells to secrete specific cytokines has led to major advances in tumor immunity.

The theoretical background of genetically modified tumor vaccines based on immunotherapy is that the host possesses antigens that can recognize tumors as external factors. Human T and B lymphocytes have the ability to discriminate almost infinite antigen differences in the form of antigen receptors through the development process. However, in order to actually succeed in tumor immunity, the following two criteria must be met. First, tumor cells must express novel antigens (peptides) that are not expressed in normal cells. Second, immune cells must be properly activated to recognize these antigens.

The conventional immunotherapy using tumor cells introduced with cytokine genes showed its effectiveness in animal experiments using white mice. Currently, studies are being conducted around the world that when tumor cells introduced with specific cytokine genes are injected into white papers, new tumors can be eradicated and tumor immunity can be acquired in these white mice.

Globally, more than 10 million people are diagnosed with cancer each year, and this number will increase to 15 million new cases annually by 2020. Cancer causes 6 million deaths each year, or 12% of deaths worldwide. There remains a need for methods that can treat cancer. These methods can provide a basis for pharmaceutical compositions useful for the prevention or treatment of cancer in humans and other mammals.

In particular, co-administration for the treatment of cancer is becoming more and more common as the benefit of attacking the disease through multiple means is recognized. Co-administration is useful even when resistance to anticancer drugs is shown. In addition, co-administration has the advantage of reducing the amount of the anticancer agent administered by enhancing the efficacy of the anticancer agent. Through this, it is possible to increase the anticancer efficacy while minimizing the toxicity and side effects on each organ of the body. Although a number of effective combination therapies have been identified over the past few decades; in view of the continuing high annual cancer deaths, there is a continuing need to identify effective therapies for use in anticancer therapies.

Patent reference 1 discloses a pharmaceutical composition for use in co-administration for the prevention or treatment of cancer, comprising a p53 activator and a c-Met inhibitor as active ingredients.

Lee Keunho et al (Biochemical and Biophysical Research Communications, Elsevier, Amsterdam NL, vol. 450, no. 4, 5 July 2014, pages 1320-1326) discloses combined effect of vascular-leakage-blocker Sac-1004 and antiangiogenic drug sunitinib on tumor angiogenesis.

### SUMMARY OF THE INVENTION

The invention is defined by the claims. Any subject-matter falling outside the scope of the claims is provided for information purposes only. Any references in the description to methods of treatment refer to the pharmaceutical composition and medicament according to the present invention for use in a method for treatment by therapy.

It is an object of the present invention to provide a cancer immunotherapy adjuvant, as defined in the claims.

It is another object of the present invention to provide a combination drug for cancer immunotherapy comprising a cancer immunotherapy agent; and a cancer immunotherapy adjuvant, as defined in the claims.

It is another object of the present disclosure to provide a pharmaceutical composition for use in enhancing the efficacy of a cancer immunotherapy agent.

It is another object of the present disclosure to provide a pharmaceutical composition for use in enhancing immunity.

It is another object of the present disclosure to provide a method for preventing or treating cancer (not part of the invention).

It is another object of the present invention to provide a kit for anticancer treatment, as defined in the claims.

To achieve the above objects, in an aspect of the present disclosure, the present disclosure provides a cancer immunotherapy adjuvant comprising a compound represented by formula 1 below, an isomer thereof, a solvate thereof, a hydrate thereof, or a pharmaceutically acceptable salt thereof as an active ingredient. 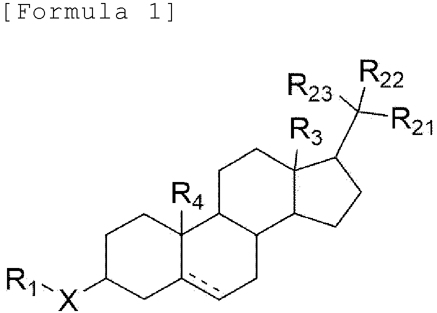 (In formula 1,
X is oxygen or sulfur; 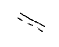 represents single bond or double bond;
R₁ is hydrogen, halo, C₁₋₃₀ alkyl, C₃₋₁₀ cycloalkyl, C₂₋₃₀ alkenyl, C₃₋₁₀ cycloalkenyl, C₂₋₁₅ heterocycloalkyl containing oxygen, sulfur or nitrogen as a heteroatom, C₃₋₁₅ heterocycloalkyl containing oxygen, sulfur or nitrogen as a heteroatom, C₂₋₃₀ alkoxyalkyl, C₃₋₃₀ alkoxyalkyl, C₃₋₁₀ heterocycloalkenyl containing oxygen, sulfur or nitrogen as a heteroatom, C₁₋₂₀ alcohol, C₁₋₂₀ alkenol, C₂₋₃₀ acyl, C₁₋₁₀ amide, C₁₋₁₀ amine, C₂₋₁₅ ester, sulfate, carboxyl group, C₃₋₂₀ carboxyalkyl, C₃₋₂₀ carboxyalkenyl, C₃₋₂₀ alkylcarboxyl, C₃₋₂₀ alkenylcarboxyl, C₃₋₂₀ alkylcarboxyalkyl, C₃₋₂₀ alkylcarboxyalkenyl, C₃₋₂₀ alkenylcarboxyalkyl, C₄₋₂₀ alkenylcarboxyalkenyl, C₆₋₃₀ aryl, C₆₋₃₀ aralkyl, C₆₋₃₀ alkaryl, C₃₋₃₀ heteroaryl or C₆₋₃₀ arylcarbonyl containing nitrogen as a heteroatom;
R₂₁ is C₂₋₃₀ alkyl, C₃₋₁₀ cycloalkyl, C₂₋₃₀ alkenyl, C₃₋₁₀ cycloalkenyl, C₂₋₃₀ carboxyalkyl, C₂₋₃₀ alkylcarboxyl, C₃₋₃₀ carboxyalkenyl, C₃₋₃₀ alkenylcarboxyl, C₃₋₃₀ alkylcarboxyalkyl, C₃₋₃₀ alkylcarboxyalkenyl, C₃₋₃₀ alkenylcarboxyalkyl, C₄₋₃₀ alkenylcarboxyalkenyl, C₂₋₁₀ heterocycloalkyl containing oxygen, sulfur or nitrogen as a heteroatom, C₃₋₁₀ heterocycloalkyl containing oxygen, sulfur or nitrogen as a heteroatom, C₂₋₃₀ alkoxyalkyl, C₃₋₃₀ alkoxyalkyl, C₃₋₁₀ heterocycloalkenyl containing oxygen, sulfur or nitrogen as a heteroatom, C₁₋₂₀ alcohol, C₁₋₂₀ alkenol, C₂₋₃₀ acyl, C₁₋₁₀ amide, C₁₋₁₀ amine or C₂₋₁₅ ester;
R₂₂ is hydrogen, hydroxy, halo or C₁₋₁₀ alkyl;
R₂₃ is hydrogen, hydroxyl or C₁₋₁₀ alkyl;
R₂₁ may form double bond to the carbon bonded together with R₂₂ and R₂₃;
R₂₃ may form double bond to the carbon bonded together with R₂₁ and R₂₂;
when R₂₁ or R₂₃ forms double bond to the carbon, R₂₂ contains no atoms; and
R₃ and R₄ are independently hydrogen or C₁₋₁₀ alkyl).

In another aspect of the present invention, the present invention provides a combination drug for cancer immunotherapy comprising a cancer immunotherapy agent; and a cancer immunotherapy adjuvant.

In another aspect of the present disclosure, the present disclosure provides a pharmaceutical composition for use in enhancing the efficacy of a cancer immunotherapy agent comprising a compound represented by formula 1, an isomer thereof, a solvate thereof, a hydrate thereof, or a pharmaceutically acceptable salt thereof as an active ingredient.

In another aspect of the present disclosure, the present disclosure provides a pharmaceutical composition for use in enhancing immunity comprising a compound represented by formula 1, an isomer thereof, a solvate thereof, a hydrate thereof, or a pharmaceutically acceptable salt thereof as an active ingredient.

In another aspect of the present disclosure, the present disclosure provides a method for preventing or treating cancer comprising a step of administering a cancer immunotherapy agent; and a cancer immunotherapy adjuvant to a subject in need thereof (not part of the invention).

In another aspect of the present invention, the present invention provides a kit for anticancer treatment comprising a cancer immunotherapy agent; and a cancer immunotherapy adjuvant as active ingredients.

### ADVANTAGEOUS EFFECT

The cancer immunotherapy adjuvant according to the present invention, when administered in combination with a cancer immunotherapy agent, activates the function of immune factors without causing in vivo side effects, to exhibit the effect of enhancing the kit for anticancer effect of the cancer immunotherapy agent, and thus can be effectively used as a cancer immunotherapy adjuvant.

### BRIEF DESCRIPTION OF THE DRAWINGS

Figure 1a is a schematic diagram showing the procedure from the preparation of an experimental animal model to the sacrifice of the animal model.
Figure 1b is a graph showing the changes in tumor size in mice according to the MC38 colorectal cancer cell line injection and drug administration.
Figure 1c is a graph showing the survival rate of mice according to the MC38 colorectal cancer cell line injection and drug administration.
Figure 2a is a photograph of a mouse before the excision of the tumor and spleen after the MC38 colorectal cancer cell line injection and drug administration.
Figure 2b is a photograph of the spleen extracted from the mouse injected with MC38 colorectal cancer cell line and administered with drugs.
Figure 2c is a photograph of the tumor extracted from the mouse injected with MC38 colorectal cancer cell line and administered with drugs.
Figure 2d is a graph showing the changes in the weight of the mouse injected with MC38 colorectal cancer cell line and administered with drugs.
Figure 2e is a graph showing the tumor weight of the mouse injected with MC38 colorectal cancer cell line and administered with drugs.
Figure 2f is a graph showing the changes in the spleen weight of the mouse injected with MC38 colorectal cancer cell line and administered with drugs.
Figures 3a to 3c are the results of FACS of the tumor of the mouse injected with MC38 colorectal cancer cell line and administered with drugs.
Figure 3d is a graph showing the level of immune factors in each drug administration group through the CD45.2+ marker.
Figure 3e is a graph showing the level of CD4⁺T cells in each drug administration group.
Figure 3f is a graph showing the level of CD8⁺T cells in each drug administration group.
Figure 3g is a graph showing the level of natural killer cells in each drug administration group.
Figure 3h is a graph showing the level of regulatory T cells in each drug administration group.
Figure 4a is a graph showing the proliferative capacity of CD4⁺T cells in each drug administration group as total%.
Figure 4b is a graph showing the proliferative capacity of CD8⁺T cells in each drug administration group as total%.
Figure 4c is a graph showing the proliferative capacity of natural miller cells in each drug administration group as total%.
Figure 4d is a graph showing the proliferative capacity of CD4⁺T cells in each drug administration group as MFI (mean fluorescence intensity).
Figure 4e is a graph showing the proliferative capacity of CD8⁺T cells in each drug administration group as MFI (mean fluorescence intensity).
Figure 4f is a graph showing the proliferative capacity of natural killer cells in each drug administration group as MFI (mean fluorescence intensity).
Figure 5a is a graph showing the level of CD107a in CD4⁺T cells in each drug administration group.
Figure 5b is a graph showing the level of CD107a in CD8⁺T cells in each drug administration group.
Figure 6a is a graph showing the level of TNFα in CD4⁺T cells in each drug administration group.
Figure 6b is a graph showing the level of TNFα in CD8⁺T cells in each drug administration group.
Figure 7a is a graph showing the level of IFNγ in CD4⁺T cells in each drug administration group.
Figure 7b is a graph showing the level of IFNγ in CD8⁺T cells in each drug administration group.
Figures 8a and 8b are the results of FACS of the tumor of the mouse injected with MC38 colorectal cancer cell line and administered with drugs.
Figure 8c is a graph showing the CD107a level of CD4⁺T cells in the spleen of each drug administration group.
Figure 8d is a graph showing the TNFα level of CD4⁺T cells in the spleen of each drug administration group.
Figure 8e is a graph showing the IFNγ level of CD4⁺T cells in the spleen of each drug administration group.
Figure 8f is a graph showing the CD107a level of CD8⁺T cells in the spleen of each drug administration group.
Figure 8g is a graph showing the TNFα level of CD8⁺T cells in the spleen of each drug administration group.
Figure 8h is a graph showing the IFNγ level of CD8⁺T cells in the spleen of each drug administration group.
Figure 9a is a fluorescence photograph showing the expression level of intratumoral adherent junction in each drug administration group.
Figure 9b is a graph showing the fluorescence density by quantifying the expression level of intratumoral adherent junction in each drug administration group.
Figure 10a is a fluorescence photograph showing the expression levels of PDL1 and CD3 in each drug administration group.
Figure 11a is a photograph showing the results of RT-PCR for the expressions of pro-inflammatory cytokines and anti-inflammatory cytokines in each drug administration group.
Figure 11b is a photograph showing the results of RT-PCR for the expressions of CXCL9, iNOS and Gapdh in each drug administration group.
Figure 11c is a photograph showing the mRNA expression level graphically through RT-PCR for each group.
Figure 12a is a schematic diagram showing the procedure according to CD4/8+T, NK removal, MC38 colorectal cancer cell line injection and drug administration.
Figure 12b is a diagram confirming that the results according to CD4/8+T, NK removal, MC38 colorectal cancer cell line injection and drug administration were confirmed through flow cytometry, and that the removal of immune cells proceeded smoothly.
Figure 12c is a diagram showing the survival rate of the mouse according to CD4/8+T and NK removal after the MC38 colorectal cancer cell line injection and drug administration, and confirming that the experimental group mouse in which CD8+ T cells were removed had the lowest survival rate.
Figure 12d is a diagram illustrating the same process as Figure 12c, showing the growth rate of the tumor according to the CD4/8+T removal over time after the MC38 colorectal cancer cell line injection and drug administration, and confirming that the tumor growth rate was the highest in the experimental group mouse in which CD8+ T cells were removed.
Figure 12e is a diagram showing the comparison of the tumor size according to the CD4/8+T and NK removal in each experimental group after the MC38 colorectal cancer cell line injection and drug administration, and confirming that the tumor size was the largest in the experimental group mouse in which CD8+ T cells were removed.
Figure 13a is a schematic diagram showing the procedure of the MC38 colorectal cancer cell line injection and long-term drug administration.
Figure 13b is a graph showing the survival rate of the mouse according to the MC38 colorectal cancer cell line injection and long-term drug administration.
Figure 13c is a graph showing the tumor size in the mouse according to the MC38 colorectal cancer cell line injection and long-term drug administration.

### DESCRIPTION OF THE PREFERRED EMBODIMENTS

Hereinafter, the present invention is described in detail.

It is well understood by those in the art who has the average knowledge on this field that the embodiments of the present invention are given to explain the present invention more precisely. In addition, the "inclusion" of an element throughout the specification does not exclude other elements, but may include other elements, unless specifically stated otherwise.

In an aspect of the present invention, the present invention provides a cancer immunotherapy adjuvant.

Particularly, the present disclosure provides a cancer immunotherapy adjuvant comprising a compound represented by formula 1 below, an isomer thereof, a solvate thereof, a hydrate thereof, or a pharmaceutically acceptable salt thereof as an active ingredient. 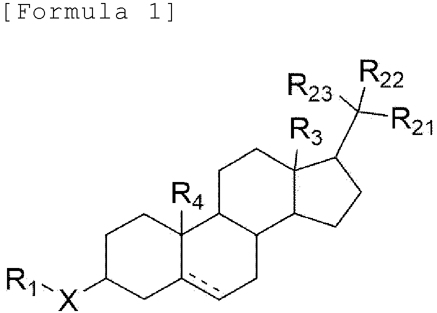 (In formula 1,
X is oxygen or sulfur; 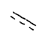 represents single bond or double bond;
R₁ is hydrogen, halo, C₁₋₃₀ alkyl, C₃₋₁₀ cycloalkyl, C₂₋₃₀ alkenyl, C₃₋₁₀ cycloalkenyl, C₂₋₁₅ heterocycloalkyl containing oxygen, sulfur or nitrogen as a heteroatom, C₃₋₁₅ heterocycloalkyl containing oxygen, sulfur or nitrogen as a heteroatom, C₂₋₃₀ alkoxyalkyl, C₃₋₃₀ alkoxyalkyl, C₃₋₁₀ heterocycloalkenyl containing oxygen, sulfur or nitrogen as a heteroatom, C₁₋₂₀ alcohol, C₁₋₂₀ alkenol, C₂₋₃₀ acyl, C₁₋₁₀ amide, C₁₋₁₀ amine, C₂₋₁₅ ester, sulfate, carboxyl group, C₃₋₂₀ carboxyalkyl, C₃₋₂₀ carboxyalkenyl, C₃₋₂₀ alkylcarboxyl, C₃₋₂₀ alkenylcarboxyl, C₃₋₂₀ alkylcarboxyalkyl, C₃₋₂₀ alkylcarboxyalkenyl, C₃₋₂₀ alkenylcarboxyalkyl, C₄₋₂₀ alkenylcarboxyalkenyl, C₆₋₃₀ aryl, C₆₋₃₀ aralkyl, C₆₋₃₀ alkaryl, C₃₋₃₀ heteroaryl or C₆₋₃₀ arylcarbonyl containing nitrogen as a heteroatom;
R₂₁ is C₂₋₃₀ alkyl, C₃₋₁₀ cycloalkyl, C₂₋₃₀ alkenyl, C₃₋₁₀ cycloalkenyl, C₂₋₃₀ carboxyalkyl, C₂₋₃₀ alkylcarboxyl, C₃₋₃₀ carboxyalkenyl, C₃₋₃₀ alkenylcarboxyl, C₃₋₃₀ alkylcarboxyalkyl, C₃₋₃₀ alkylcarboxyalkenyl, C₃₋₃₀ alkenylcarboxyalkyl, C₄₋₃₀ alkenylcarboxyalkenyl, C₂₋₁₀ heterocycloalkyl containing oxygen, sulfur or nitrogen as a heteroatom, C₃₋₁₀ heterocycloalkyl containing oxygen, sulfur or nitrogen as a heteroatom, C₂₋₃₀ alkoxyalkyl, C₃₋₃₀ alkoxyalkyl, C₃₋₁₀ heterocycloalkenyl containing oxygen, sulfur or nitrogen as a heteroatom, C₁₋₂₀ alcohol, C₁₋₂₀ alkenol, C₂₋₃₀ acyl, C₁₋₁₀ amide, C₁₋₁₀ amine or C₂₋₁₅ ester;
R₂₂ is hydrogen, hydroxy, halo or C₁₋₁₀ alkyl;
R₂₃ is hydrogen, hydroxyl or C₁₋₁₀ alkyl;
R₂₁ may form double bond to the carbon bonded together with R₂₂ and R₂₃;
R₂₃ may form double bond to the carbon bonded together with R₂₁ and R₂₂;
when R₂₁ or R₂₃ forms double bond to the carbon, R₂₂ contains no atoms; and
R₃ and R₄ are independently hydrogen or C₁₋₁₀ alkyl).
X in formula 1 may be oxygen.
R₁ in formula 1 may be hydrogen, halo, C₁₋₁₀ alkyl, C₃₋₈ cycloalkyl, C₂₋₁₀ alkenyl, C₃₋₈ cycloalkenyl, C₂₋₈ heterocycloalkyl containing oxygen, sulfur or nitrogen as a heteroatom, C₃₋₁₀ heterocycloalkyl containing oxygen, sulfur or nitrogen as a heteroatom, C₂₋₂₀ alkoxyalkyl, C₃₋₂₀ alkoxyalkyl, C₃₋₈ heterocycloalkenyl containing oxygen, sulfur or nitrogen as a heteroatom, C₁₋₁₀ alcohol, C₁₋₁₀ alkenol, C₂₋₂₀ acyl, C₁₋₁₀ amide, C₁₋₅ amine, C₂₋₁₅ ester, sulfate, carboxyl group, C₃₋₂₀ carboxyalkyl, C₃₋₂₀ carboxyalkenyl, C₃₋₂₀ alkylcarboxyl, C₃₋₂₀ alkenylcarboxyl, C₃₋₂₀ alkylcarboxyalkyl, C₃₋₂₀ alkylcarboxyalkenyl, C₃₋₂₀ alkenylcarboxyalkyl, C₄₋₂₀ alkenylcarboxyalkenyl, C₆₋₂₀ aryl, C₆₋₂₀ aralkyl, C₆₋₂₀ alkaryl, C₃₋₂₀ heteroaryl or C₆₋₂₀ arylcarbonyl containing nitrogen as a heteroatom.
R₁ in formula 1 may be hydrogen, C₁₋₁₀ alkyl, C₃₋₈ cycloalkyl, C₂₋₁₀ alkenyl, C₃₋₈ cycloalkenyl, C₂₋₈ heterocycloalkyl containing oxygen as a heteroatom, C₃₋₁₀ heterocycloalkyl containing oxygen as a heteroatom, C₂₋₂₀ alkoxyalkyl, C₃₋₁₀ alkoxyalkyl, C₃₋₈ heterocycloalkenyl containing oxygen as a heteroatom, C₁₋₁₀ alcohol, C₁₋₁₀ alkenol, C₁₋₁₀ amide, C₁₋₅ amine, C₂₋₁₅ ester, sulfate, carboxyl group, C₃₋₂₀ carboxyalkyl, C₃₋₂₀ carboxyalkenyl, C₃₋₂₀ alkylcarboxyl, C₃₋₂₀ alkenylcarboxyl, C₃₋₂₀ alkylcarboxyalkyl, C₃₋₂₀ alkylcarboxyalkenyl, C₃₋₂₀ alkenylcarboxyalkyl, C₄₋₂₀ alkenylcarboxyalkenyl, C₆₋₂₀ aryl, C₆₋₂₀ aralkyl, C₆₋₂₀ alkaryl, C₃₋₂₀ heteroaryl or C₆₋₂₀ arylcarbonyl containing nitrogen as a heteroatom.

In R₁ of formula 1, cycloalkyl or heterocycloalkyl can be substituted with hydroxy, halo, C₁₋₅ alkyl, C₁₋₅ alcohol, C₁₋₅ alkoxy, C₂₋₈ alkoxyalkyl, C₆₋₂₀ aryl, C₇₋₂₀ arylcarboxyl or a combination thereof; C₃₋₁₀ cycloalkenyl or heterocycloalkenyl can be substituted with hydroxy, halo, C₁₋₅ alkyl, C₂₋₈ alkylcarboxyl, C₃₋₈ alkylcarboxylalkyl, C₁₋₅ alcohol, C₁₋₅ alkoxy, C₂₋₈ alkoxyalkyl, C₆₋₂₀ aryl, C₇₋₂₀ arylcarboxyl or a combination thereof; aryl can be substituted with hydroxy, halo, C₁₋₅ alkyl, C₁₋₅ alcohol, C₁₋₅ alkoxy, C₂₋₈ alkoxyalkyl, nitro, C₂₋₈ alkylcarboxylamino or a combination thereof; aralkyl can be substituted with hydroxy, halo, C₁₋₅ alkyl, C₁₋₅ alcohol, C₁₋₅ alkoxy, C₂₋₈ alkoxyalkyl, nitro, C₂₋₈ alkylcarboxylamino or a combination thereof; alkaryl can be substituted with hydroxy, halo, C₁₋₅ alkyl, C₁₋₅ alcohol, C₁₋₅ alkoxy, C₂₋₈ alkoxyalkyl, nitro, C₂₋₈ alkylcarboxylamino or a combination thereof; arylcarbonyl can be substituted with hydroxy, halo, C₁₋₅ alkyl, C₁₋₅ alcohol, C₁₋₅ alkoxy, C₂₋₈ alkoxyalkyl, nitro, C₂₋₈ alkylcarboxylamino or a combination thereof; and heteroaryl can be substituted with hydroxy, halo, C₁₋₅ alkyl, C₁₋₅ alcohol, C₁₋₅ alkoxy, C₂₋₈ alkoxyalkyl, nitro, C₂₋₈ alkylcarboxylamino or a combination thereof.

R₂₁ in formula 1 may be straight or branched C₂₋₁₅ alkyl, C₃₋₁₀ cycloalkyl, C₂₋₁₅ alkenyl, C₃₋₁₀ cycloalkenyl, C₂₋₁₅ carboxyalkyl, C₂₋₁₅ alkylcarboxyl, C₃₋₁₅ carboxyalkenyl, C₂₋₁₅ alkenylcarboxyl, C₃₋₁₅ alkylcarboxyalkyl, C₃₋₁₅ alkylcarboxyalkenyl, C₃₋₁₅ alkenylcarboxyalkyl, C₂₋₃₀ alkenylcarboxyalkenyl, C₂₋₁₀ heterocycloalkyl containing oxygen, sulfur or nitrogen as a heteroatom, C₃₋₁₀ heterocycloalkyl containing oxygen, sulfur or nitrogen as a heteroatom, C₂₋₂₀ alkoxyalkyl, C₃₋₃₀ alkoxyalkyl, C₃₋₁₀ heterocycloalkenyl containing oxygen, sulfur or nitrogen as a heteroatom, C₁₋₂₀ alcohol, C₁₋₂₀ alkenol, C₂₋₃₀ acyl, C₁₋₁₀ amide, C₁₋₁₀ amine or C₂₋₁₅ ester.

R₂₃ in formula 1 is C₁₋₅ alkyl or may form double bond to the carbon bonded together with R₂₁ and R₂₂.

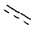 in formula 1 may be double bond.

Examples of the compound represented by formula 1 according to the present disclosure include a compound represented by formula 2 below:

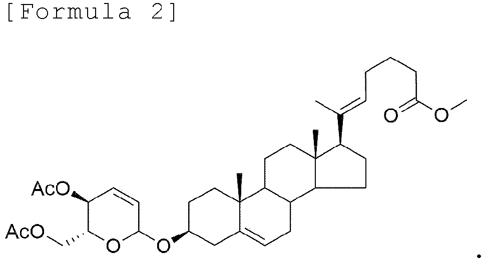

The compound represented by formula 1 of the present disclosure can be used as a form of a pharmaceutically acceptable salt, in which the salt is preferably acid addition salt formed by pharmaceutically acceptable free acids. The acid addition salt herein can be obtained from inorganic acids such as hydrochloric acid, nitric acid, phosphoric acid, sulfuric acid, hydrobromic acid, hydroiodic acid, nitrous acid, and phosphorous acid; non-toxic organic acids such as aliphatic mono and dicarboxylate, phenyl-substituted alkanoate, hydroxy alkanoate and alkandioate, aromatic acids, and aliphatic and aromatic sulfonic acids; and organic acids such as trifluoroacetic acid, acetate, benzoic acid, citric acid, lactic acid, maleic acid, gluconic acid, methanesulfonic acid, 4-toluenesulfonic acid, tartaric acid, and fumaric acid, etc. The pharmaceutically non-toxic salts are exemplified by sulfate, pyrosulfate, bisulfate, sulphite, bisulphite, nitrate, phosphate, monohydrogen phosphate, dihydrogen phosphate, metaphosphate, pyrophosphate, chloride, bromide, iodide, fluoride, acetate, propionate, decanoate, caprylate, acrylate, formate, isobutylate, caprate, heptanoate, propiolate, oxalate, malonate, succinate, suberate, cabacate, fumarate, maliate, butyne-1,4-dioate, hexane-1,6-dioate, benzoate, chlorobenzoate, methylbenzoate, dinitrobenzoate, hydroxybenzoate, methoxybenzoate, phthalate, terephthalate, benzenesulfonate, toluenesulfonate, chlorobenzenesulfonate, xylenesulfonate, phenylacetate, phenylpropionate, phenylbutylate, citrate, lactate, β-hydroxybutylate, glycolate, malate, tartrate, methanesulfonate, propanesulfonate, naphthalene-1-sulfonate, naphthalene-2-sulfonate, and mandelate.

The acid addition salt according to the present invention can be prepared by the conventional method known to those in the art. For example, the derivative represented by formula 1 is dissolved in an organic solvent such as methanol, ethanol, acetone, methylene chloride, and acetonitrile, to which organic acid or inorganic acid is added to induce precipitation. Then, the precipitate is filtered and dried to give the salt. Or the solvent and the excessive acid are distillated under reduced pressure, and dried to give the salt. Or the precipitate is crystallized in an organic solvent to give the same.

A pharmaceutically acceptable metal salt can be prepared by using a base. Alkali metal or alkali earth metal salt is obtained by the following processes: dissolving the compound in excessive alkali metal hydroxide or alkali earth metal hydroxide solution; filtering non-soluble compound salt; evaporating the remaining solution and drying thereof. At this time, the metal salt is preferably prepared in the pharmaceutically suitable form of sodium, potassium, or calcium salt. And the corresponding silver salt is prepared by the reaction of alkali metal or alkali earth metal salt with proper silver salt (ex; silver nitrate).

In addition, the present disclosure includes not only the compound represented by formula 1 but also a pharmaceutically acceptable salt thereof, and a solvate, an optical isomer, or a hydrate possibly produced from the same.

The term "hydrate" refers to the compound of the present invention or the salt thereof comprising a stoichiometric or non-stoichiometric amount of water bound by non-covalent intermolecular force. The hydrate of the compound represented by formula 1 of the present invention can include a stoichiometric or non-stoichiometric amount of water that is bound by non-covalent intermolecular force. The hydrate can contain more than 1 equivalent of water, preferably, 1 to 5 equivalents of water. Such a hydrate can be prepared by crystallizing the compound represented by formula 1 of the present invention, the isomer thereof, or the pharmaceutically acceptable salt thereof from water or a solvent containing water.

The term "solvate" refers to the compound of the present invention or the salt thereof comprising a stoichiometric or non-stoichiometric amount of a solvent bound by non-covalent intermolecular force. Preferred solvents therefor include solvents that are volatile, non-toxic, and/or suitable for administration to humans.

The term "isomer" refers to the compound of the present invention or the salt thereof having the same chemical or molecular formula but different structurally or sterically. Such isomers include structural isomers such as tautomers, stereoisomers such as geometric isomers (trans, cis), and optical isomers (enantiomers). Of these, only stereoisomers are included in the scope of the present invention.

In the cancer immunotherapy adjuvant according to the present disclosure, the compound represented by formula 1 or the pharmaceutically acceptable salt thereof can be administered orally or parenterally in various formulations at the time of clinical administration. More preferably, they can be parenteral formulations. The compound represented by formula 1 or the pharmaceutically acceptable salt thereof can be prepared for oral or parenteral administration by mixing with generally used diluents or excipients such as fillers, extenders, binders, wetting agents, disintegrating agents and surfactant. Solid formulations for oral administration are tablets, pills, powders, granules and capsules. These solid formulations are prepared by mixing the compound represented by formula 1 or the pharmaceutically acceptable salt thereof of the present disclosure with one or more suitable excipients such as starch, calcium carbonate, sucrose or lactose, gelatin, etc. Except for the simple excipients, lubricants, for example magnesium stearate, talc, etc, can be used. Liquid formulations for oral administrations are suspensions, solutions, emulsions and syrups, and the above-mentioned formulations can contain various excipients such as wetting agents, sweeteners, aromatics and preservatives in addition to generally used simple diluents such as water and liquid paraffin. Formulations for parenteral administration are sterilized aqueous solutions, water-insoluble excipients, suspensions, and emulsions. Water insoluble excipients and suspensions can contain, in addition to the active compound or compounds, propylene glycol, polyethylene glycol, vegetable oil like olive oil, injectable ester like ethylolate, etc.

The cancer immunotherapy adjuvant comprising the compound represented by formula 1 or the pharmaceutically acceptable salt thereof as an active ingredient can be administered by parenterally and the parenteral administration includes subcutaneous injection, intravenous injection, intramuscular injection, or intrathoracic injection.

To prepare the compound represented by formula 1 or the pharmaceutically acceptable salt thereof as a formulation for parenteral administration, the compound represented by formula 1 or the pharmaceutically acceptable salt thereof is mixed with a stabilizer or a buffering agent in water to produce solution or suspension, which is then formulated as ampoules or vials. The composition herein can be sterilized and additionally contains preservatives, stabilizers, wettable powders or emulsifiers, salts and/or buffers for the regulation of osmotic pressure, and other therapeutically useful materials, and the composition can be formulated by the conventional mixing, granulating or coating method.

The formulations for oral administration are exemplified by tablets, pills, hard/soft capsules, solutions, suspensions, emulsions, syrups, granules, elixirs, and troches, etc. These formulations can include diluents (for example, lactose, dextrose, sucrose, mannitol, sorbitol, cellulose, and/or glycine) and lubricants (for example, silica, talc, stearate and its magnesium or calcium salt, and/or polyethylene glycol) in addition to the active ingredient. Tablets can include binding agents such as magnesium aluminum silicate, starch paste, gelatin, methylcellulose, sodium carboxymethylcellulose and/or polyvinylpyrolidone, and if necessary disintegrating agents such as starch, agarose, alginic acid or its sodium salt or azeotropic mixtures and/or absorbents, coloring agents, flavours, and sweeteners can be additionally included thereto.

The cancer immunotherapy adjuvant can enhance the efficacy of the cancer immunotherapy agent, and more specifically, it can enhance the efficacy of the cancer immunotherapy agent by activating immune factors to assist the anticancer activity of the cancer immunotherapy agent. The immune factor can be at least one selected from the group consisting of helper T cells, cytotoxic T cells, natural killer cells (NK cells), and cytokines.

The cancer immunotherapy adjuvant can be administered simultaneously or sequentially with the cancer immunotherapy agent, and when administered sequentially, the cancer immunotherapy adjuvant can be administered after the cancer immunotherapy agent is administered, or the cancer immunotherapy adjuvant can be administered after the cancer immunotherapy agent is administered. However, the administration method is only an example, and the administration method may be changed to enhance the anticancer immune effect. In an embodiment of the present disclosure, the cancer immunotherapy adjuvant was administered by intravenous injection every day, and the cancer immunotherapy agent was administered by intraperitoneal injection 3 times a week, but not always limited thereto.

The cancer immunotherapy adjuvant can activate one or more immune factors selected from the group consisting of helper T cells, cytotoxic T cells, natural killer cells (NK cells) and cytokines. The cancer immunotherapy adjuvant exhibits the effect of enhancing the anticancer effect of the cancer immunotherapy agent by activating the immune factors.

At this time, the cancer immunotherapy adjuvant can prevent or treat cancer by being administered in combination with the cancer immunotherapy agent.

The cancer can be at least one selected from the group consisting of pseudomyxoma, intrahepatic cholangiocarcinoma, hepatoblastoma, liver cancer, thyroid cancer, colon cancer, testicular cancer, myelodysplastic syndrome, glioblastoma, oral cancer, lip cancer, mycosis fungoides, acute myeloid leukemia, acute lymphoblastic leukemia, basal cell carcinoma, ovarian epithelial cancer, ovarian germ cell cancer, male breast cancer, brain cancer, pituitary adenoma, multiple myeloma, gallbladder cancer, biliary tract cancer, colorectal cancer, chronic myelogenous leukemia, chronic lymphocytic leukemia, retinoblastoma, choroidal melanoma, ampullar of vater cancer, bladder cancer, peritoneal cancer, parathyroid cancer, adrenal cancer, nasal cavity cancer, non-small cell lung cancer, tongue cancer, astrocytoma, small cell lung cancer, juvenile brain cancer, juvenile lymphoma, juvenile leukemia, small intestine cancer, meningioma, esophageal cancer, glioma, renal pelvic cancer, kidney cancer, heart cancer, duodenal cancer, malignant soft tissue cancer, malignant bone cancer, malignant lymphoma, malignant mesothelioma, malignant melanoma, eye cancer, vulvar cancer, ureter cancer, urethral cancer, cancer of unknown primary site, gastric lymphoma, stomach cancer, gastric carcinoma, gastrointestinal stromal cancer, Wilms cancer, breast cancer, triple-negative breast cancer, sarcoma, penile cancer, pharyngeal cancer, gestational villous disease, cervical cancer, endometrial cancer, uterine sarcoma, prostate cancer, metastatic bone cancer, metastatic brain cancer, mediastinal cancer, rectal cancer, rectal carcinoma, vaginal cancer, spinal cord cancer, vestibular schwannoma, pancreatic cancer, salivary gland cancer, Kaposi's sarcoma, Paget's disease, tonsil cancer, squamous cell carcinoma, lung adenocarcinoma, lung cancer, lung squamous cell carcinoma, skin cancer, anal cancer, rhabdomyosarcoma, laryngeal cancer, pleural cancer, blood cancer and thymus cancer.

The cancer immunotherapy adjuvant can be co-administered together with the conventionally known and well-known cancer immunotherapy agent to those skilled in the art without limitation. For example, the cancer immunotherapy adjuvant can be administered in combination with one or more cancer immunotherapy agents selected from the group consisting of anti-PD1, anti-PDL1, anti-CTLA4, anti-LAG3, anti-VISTA, anti-BTLA, anti-TIM3, anti-HVEM, anti-CD27, anti-CD137, anti-OX40, anti-CD28, anti-PDL2, anti-GITR, anti-ICOS, anti-SIRPα, anti-ILT2, anti-ILT3, anti-ILT4, anti-ILT5, anti-EGFR, anti-CD19 and anti-TIGIT, but not always limited thereto.

In another aspect of the present invention, the present invention provides a combination drug for cancer immunotherapy.

Particularly, the present invention provides a combination drug for cancer immunotherapy comprising a cancer immunotherapy agent and a cancer immunotherapy adjuvant.

In another aspect of the present invention, the present disclosure provides a pharmaceutical composition for use in enhancing the efficacy of a cancer immunotherapy agent.

Particularly, the present disclosure provides a pharmaceutical composition for use in enhancing the efficacy of a cancer immunotherapy agent comprising a compound represented by formula 1, an isomer thereof, a solvate thereof, a hydrate thereof, or a pharmaceutically acceptable salt thereof as an active ingredient.

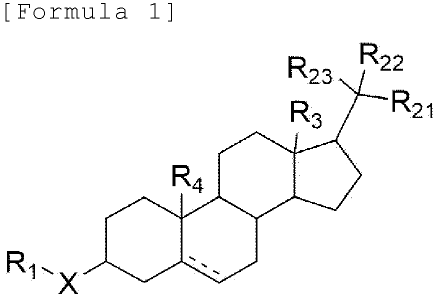

In formula 1, the specific description of R₁ to R₄ and X is the same as the specific description of formula 1 in the cancer immunotherapy adjuvant.

In addition, the specific description of the pharmaceutical composition for use in enhancing the efficacy of a cancer immunotherapy agent is the same as the specific description of the cancer immunotherapy adjuvant.

In another aspect of the present invention, the present disclosure provides a pharmaceutical composition for use in enhancing immunity.

Particularly, the present disclosure provides a pharmaceutical composition for use in enhancing immunity comprising a compound represented by formula 1, an isomer thereof, a solvate thereof, a hydrate thereof, or a pharmaceutically acceptable salt thereof as an active ingredient.

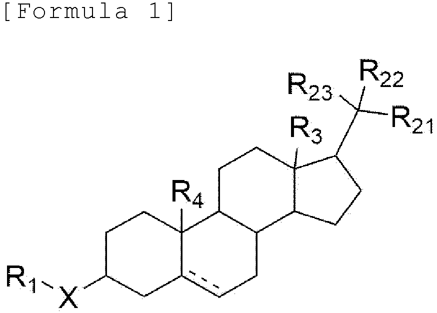

In formula 1, the specific description of R₁ to R₄ and X is the same as the specific description of formula 1 in the cancer immunotherapy adjuvant.

In addition, the specific description of the pharmaceutical composition for use in enhancing immunity is the same as the specific description of the cancer immunotherapy adjuvant.

In another aspect of the present disclosure, the present invention provides a method for preventing or treating cancer comprising a step of administering a cancer immunotherapy agent and a cancer immunotherapy adjuvant to a subject in need thereof (not part of the invention) .

The cancer immunotherapy adjuvant and the cancer immunotherapy agent can be administered in combination or at different times.

In another aspect of the present disclosure, the present invention provides a cancer immunotherapy adjuvant and an immunotherapy agent for use in the prevention or treatment of cancer.

In another aspect of the present disclosure, the present invention provides a combination therapy for the treatment of cancer comprising a step of administering a cancer immunotherapy adjuvant and a cancer immunotherapy agent to a subject in need thereof (not part of the invention).

In another aspect of the present invention, the present invention provides a kit for preventing or treating cancer comprising a cancer immunotherapy agent and a cancer immunotherapy adjuvant as an active ingredient.

Hereinafter, the present invention will be described in detail by the following examples and experimental examples.

However, the following examples and experimental examples are only for illustrating the present invention, and the contents of the present invention are not limited thereto.

### Example 1: Preparation of cancer immunotherapy adjuvant (SAC-1004)

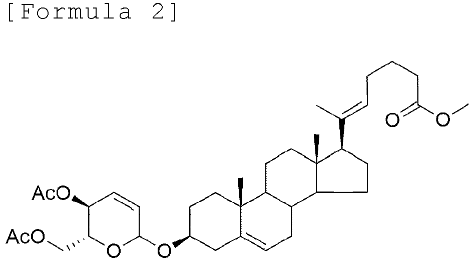

The compound SAC-1004 represented by formula 2, the pharmaceutical composition of the present invention for co-administration with a cancer immunotherapy agent, was prepared according to reaction formula 1 below based on Korean Patent Publication No. 10-2011-0047170.

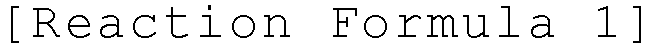

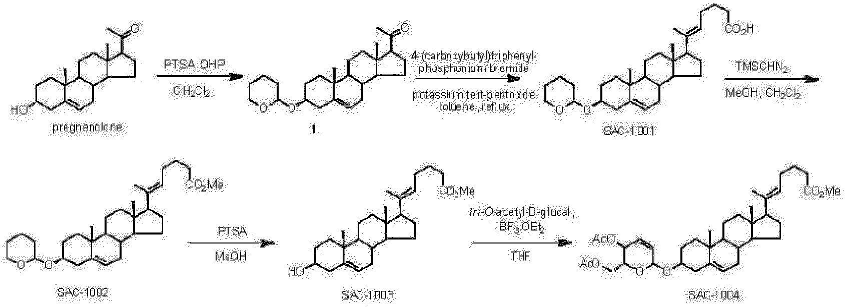

13.4 mg of SAC-1003 was dissolved in 1 mL of tetrahydrofuran, to which 26 mg of tri-O-acetyl-D-glucal (Aldrich) and 0.012 mL of borontrifluoride·diethyl etherate (Aldrich) were added under argon atmosphere, followed by stirring at 0°C for 10 hours. After raising the temperature of the reaction solution to room temperature, it was diluted by adding 5 mL of diethyl ether, washed with sodium hydrogen carbonate aqueous solution, dried over sodium sulfate, and filtered. The filtrate was concentrated under reduced pressure, and the residue was subjected to silica gel column chromatography using a mixed eluate of ethyl acetate/hexane (1:10) to give the target compound SAC-1004 (11 mg, 56%): ¹H-NMR (300MHz, CDCl₃) δ5.89-5.80 (m, 2H), 5.37-5.27 (m, 2H), 5.17-5.14 (m, 2H), 4.23-4.16 (m, 3H), 3.66 (s, 3H), 3.56 (m, 1H), 2.38-2.28 (m, 4H), 2.17-0.53 (m, 37H).

### <Experimental Method>

### 1. Preparation of experimental animal model

MC-38 colorectal cancer cells (5x10⁵ cells) were injected subcutaneously into 7-week-old C57BL/6 male mice. Seven days after the colorectal cancer cells were injected, the average tumor volume of the subcutaneous MC-38 tumor was about 40 mm³. The injection method of the mouse tumor as described above was equally applied to all four groups below. Afterwards, the experimental group was divided into the vehicle administration control group, SAC-1004 and anti-PD1 co-administration group, SAC-1004 single administration group, and anti-PD1 single administration group, and the experiment was performed.

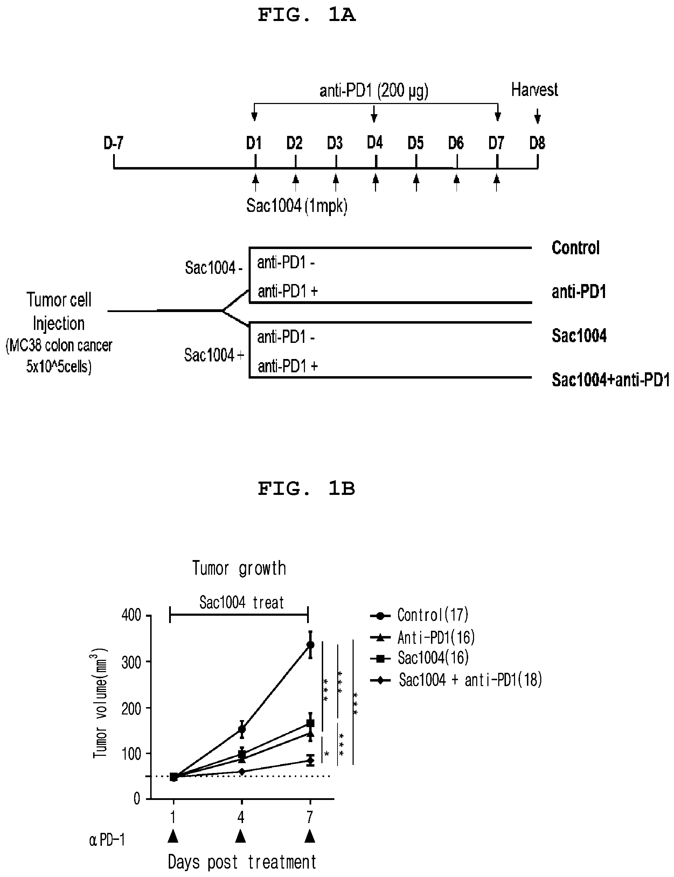

Figure 1a is a schematic diagram showing the procedure from the preparation of an experimental animal model to the sacrifice of the animal model.

Specifically, the administration was performed as follows. SAC-1004, a vascular leak inhibitor, was dissolved in DMSO in PBS, and 1 mg per kg of mouse body weight was administered daily by intravenous injection. Anti-PD1, an immune checkpoint inhibitor, was administered at the concentration of 200 ng/mouse by intraperitoneal injection 3 times a week along with 200 ng of rat igG2a. As described above, SAC-1004 and anti-PD1 were administered to each group for 7 days, and the tumor and the spleen were extracted on the 8^{th} day.

### 2. RNA isolation, cDNA synthesis and PCR analysis

Total RNA was isolated from the extracted tumor tissues using Trizol reagent. The concentration of the isolated RNA was determined by measuring the absorbance at 260/280 nm using nano-drop (ND-1000, Thermo scientific), and cDNA was synthesized by including 2 µg of RNA and making the total volume to 20 µl.

### 3. Fluorescence-activated cell sorting (FACS) of mouse tumor

After separating cells from the tumor tissue using collagenase, a medium was added to the cells, centrifuged, and the precipitated cells were collected and counted. Living cells were stained at 37°C for about 30 minutes. After staining only living cells, followed by washing with PBS three times. Calibration was conducted through live/dead staining. In addition, FACS was performed using Canto. Through this, the activity ratio of T cells could be compared.

T cells are involved in adaptive immune response and are divided into helper T cells (CD4⁺T cells), cytotoxic T cells (CD8⁺T cells), and regulatory T cells (Treg cells) derived from CD4⁺T cells but suppressing cytotoxic T cells. In addition, there are natural killer cells (NK cells) with cytotoxic properties involved in innate immune response, so the levels of these cells in each group were measured.

CD4 antibody was used as a marker for measuring CD4⁺T cells, which are helper T cells, and CD8 antibody was used as a marker for measuring CD8⁺T cells, which are cytotoxic T cells. In addition, CD25 antibody and Foxp³ antibody were used to measure regulatory T cells, and NK1.1 antibody was used to measure natural killer cells.

### 4. IHC staining

The tumor tissue was immediately taken out and then O/N incubated in 4% PFA (stored at 4°C) . After the tumor tissue was submerged sequentially in 15% to 30% sucrose to sink, OCT sampling was performed on dry ice. 20 µm sections were stained with each primary antibody and secondary antibody.

### 5. Analysis of T cell function

The function of T cells is mainly measured in two ways. One method is cytokine production capacity analysis, which measures the secretion of IFNγ or TNFα, which are pro-inflammatory cytokines, from T cells. Another method is a method of measuring cytotoxicity, in which tumor cells are directly ligated and killed by inserting perforin or granzyme B into the tumor cells.

### Experimental Example 1: Analysis of tumor size change in mice according to MC38 colorectal cancer cell line injection and drug administration

In a total of 4 groups consisting of control group, SAC-1004 and anti-PD1 co-administration group, SAC-1004 single administration group, and anti-PD1 single administration group, the tumor size changes according to the drug administration for 7 days as described above in the experimental animal model were analyzed.

Figure 1b is a graph showing the changes in tumor size in mice according to the MC38 colorectal cancer cell line injection and drug administration.

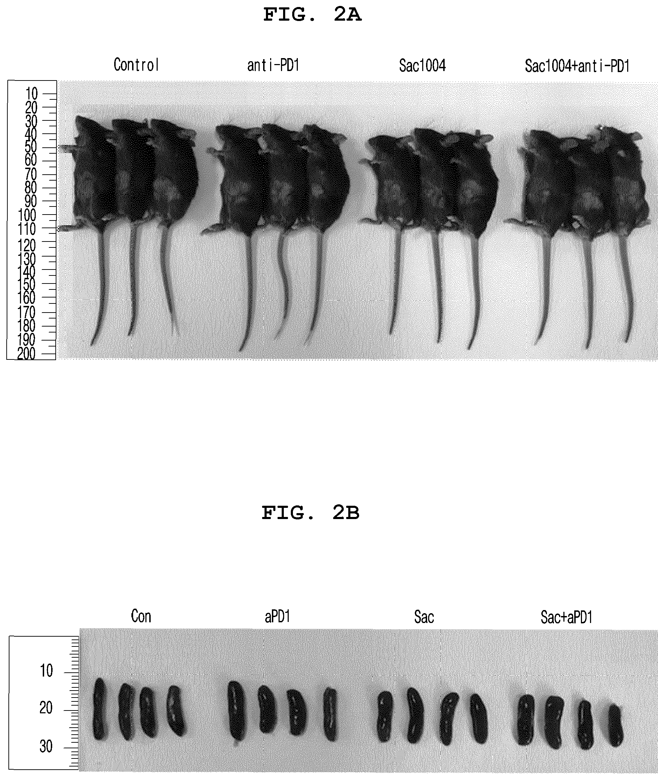

Figure 2a is a photograph of a mouse before the excision of the tumor and spleen after the MC38 colorectal cancer cell line injection and drug administration.

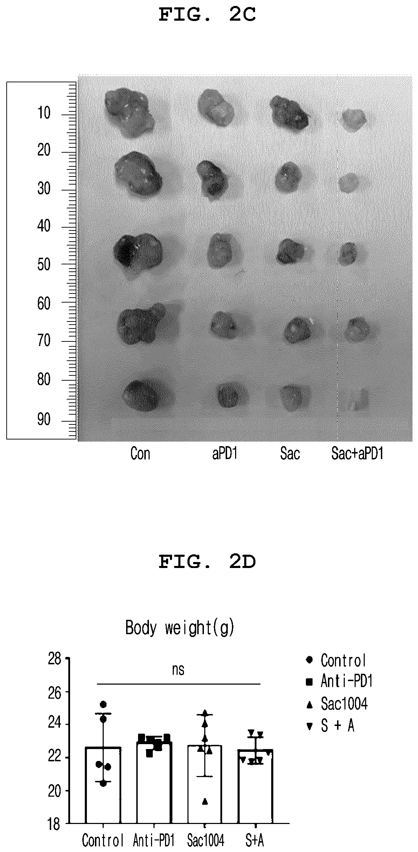

Figure 2c is a photograph of the tumor extracted from the mouse injected with MC38 colorectal cancer cell line and administered with drugs.

Particularly, the tumor size in the anti-PD1 single administration group and the SAC-1004 single administration group was reduced compared to that in the control group, and the tumor size in the SAC-1004 and anti-PD1 co-administration group was decreased compared to those in the single administration groups. The results were statistically proved by two-way ANOVA.

The above results demonstrate that the preventive or therapeutic effect on cancer was significantly increased when SAC-1004 and the immunotherapy agent were administered in combination rather than when administered alone.

### Experimental Example 2: Analysis of survival rate of mice according to MC38 colorectal cancer cell line injection and drug administration

In a total of 4 groups consisting of control group, SAC-1004 and anti-PD1 co-administration group, SAC-1004 single administration group, and anti-PD1 single administration group, the survival rate of mice according to the drug administration for 7 days as described above in the experimental animal model was analyzed.

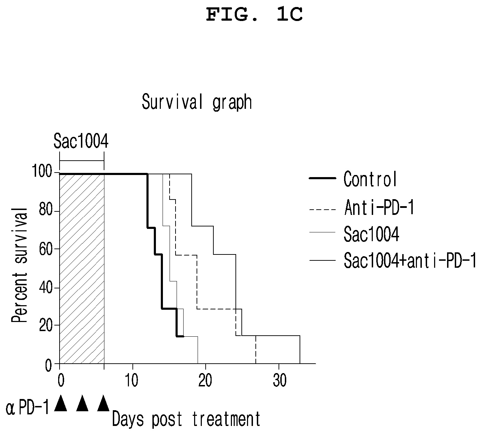

Figure 1c is a graph showing the survival rate of mice according to the MC38 colorectal cancer cell line injection and drug administration.

Particularly, the survival rate of mice according to the drug administration in the anti-PD1 single administration group was higher than that in the control group or the SAC-1004 single administration group, and the survival rate of mice in the SAC-1004 and anti-PD1 co-administration group was higher than that in the anti-PD1 single administration group. The results were statistically proved by two-way ANOVA.

The above results demonstrate that the preventive or therapeutic effect on cancer was significantly increased when SAC-1004 and the immunotherapy agent were administered in combination rather than when administered alone.

### Experimental Example 3: Analysis of weight changes in mice according to MC38 colorectal cancer cell line injection and drug administration

In a total of 4 groups consisting of control group, SAC-1004 and anti-PD1 co-administration group, SAC-1004 single administration group, and anti-PD1 single administration group, the weight changes in mice according to the drug administration for 7 days as described above in the experimental animal model were analyzed.

Figure 2d is a graph showing the changes in the weight of the mouse injected with MC38 colorectal cancer cell line and administered with drugs.

Particularly, similar body weights were measured in all groups, indicating that no abnormalities were observed in mice even after long-term administration of the drug.

The above results demonstrate that the pharmaceutical composition for use in co-administration according to the present invention did not cause side effects *in vivo* even when administered alone or in combination, and is a material with safety secured.

### Experimental Example 4: Comparison of spleen size and weight of mice injected with MC38 colorectal cancer cell line and administered with drugs

In a total of 4 groups consisting of control group, SAC-1004 and anti-PD1 co-administration group, SAC-1004 single administration group, and anti-PD1 single administration group, the spleen size of mice treated with the drug for 7 days as described above in the experimental animal model was analyzed.

Figure 2b is a photograph of the spleen extracted from the mouse injected with MC38 colorectal cancer cell line and administered with drugs.

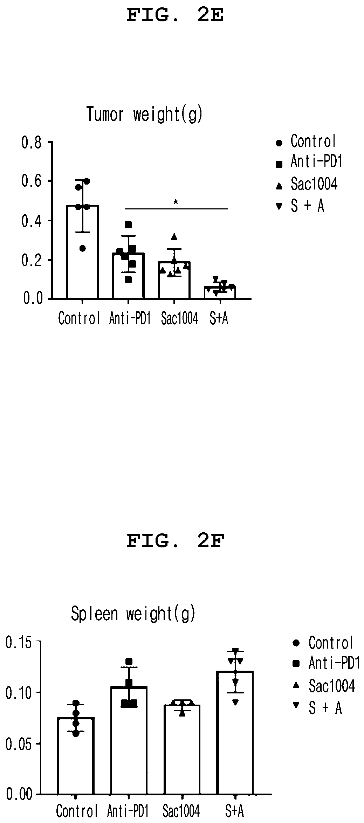

Figure 2f is a graph showing the changes in the spleen weight of the mouse injected with MC38 colorectal cancer cell line and administered with drugs.

Particularly, the spleen weight of the SAC-1004 single administration group mice was higher than that of the control group mice, the spleen weight of the anti-PD1 single administration group mice was higher than that of the SAC-1004 single administration group mice, and the spleen weight of the SAC-1004 and anti-PD1 co-administration group mice was higher than that of the anti-PD1 single administration group mice. However, this was not statistically significant when verified by one-way ANOVA.

The above results demonstrate that the pharmaceutical composition for use in co-administration according to the present invention did not cause side effects *in vivo* even when administered alone or in combination, and is a material with safety secured.

### Experimental Example 5: Comparison of tumor weight of mice injected with MC38 colorectal cancer cell line and administered with drugs

In a total of 4 groups consisting of control group, SAC-1004 and anti-PD1 co-administration group, SAC-1004 single administration group, and anti-PD1 single administration group, the tumor weight of mice treated with the drug for 7 days as described above in the experimental animal model was analyzed.

Figure 2e is a graph showing the tumor weight of the mouse injected with MC38 colorectal cancer cell line and administered with drugs.

Particularly, the tumor weight of the anti-PD1 single administration group mice was lower than that of the control group mice, the tumor weight of the SAC-1004 single administration group mice was lower than that of the anti-PD1 single administration group mice, and the tumor weight of the SAC-1004 and anti-PD1 co-administration group mice was lower than that of the SAC-1004 single administration group mice. The results were statistically proved by one-way ANOVA.

The above results demonstrate that the preventive or therapeutic effect on cancer was significantly increased when SAC-1004 and the cancer immunotherapy agent were administered in combination rather than when administered alone.

### Experimental Example 6: FACS analysis of mouse tumor

In a total of 4 groups consisting of control group, SAC-1004 and anti-PD1 co-administration group, SAC-1004 single administration group, and anti-PD1 single administration group, FACS (fluorescence activated cell sorting) analysis was performed on the tumors of mice treated with the drug for 7 days as described above in the experimental animal model, and the activity ratio of T cells in each drug-administered group was compared.

CD4 antibody was used to measure CD4⁺T cells, which are helper T cells, and CD8 antibody was used to measure CD8⁺T cells, which are cytotoxic T cells. In addition, CD25 antibody and Foxp³ antibody were used to measure regulatory T cells, and NK1.1 antibody was used to measure natural killer cells.

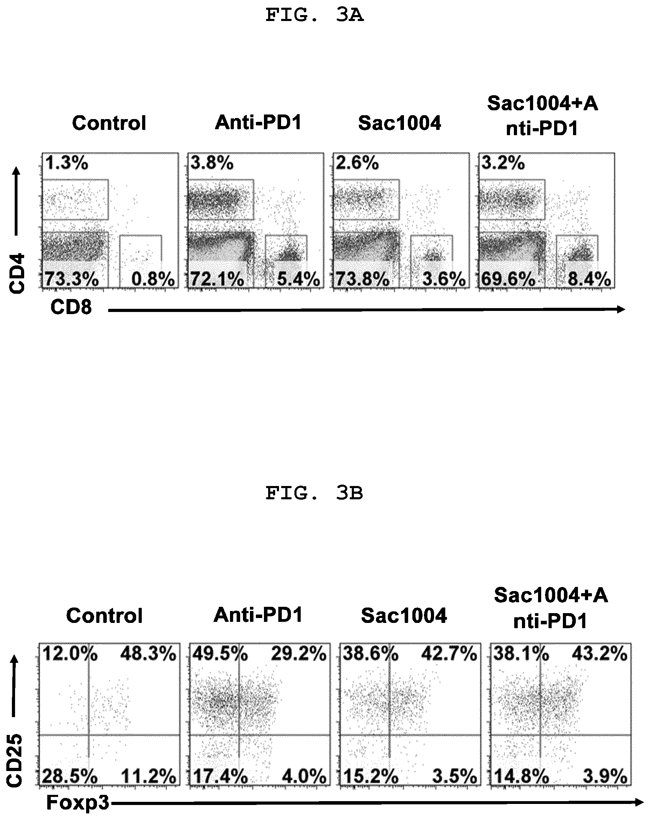

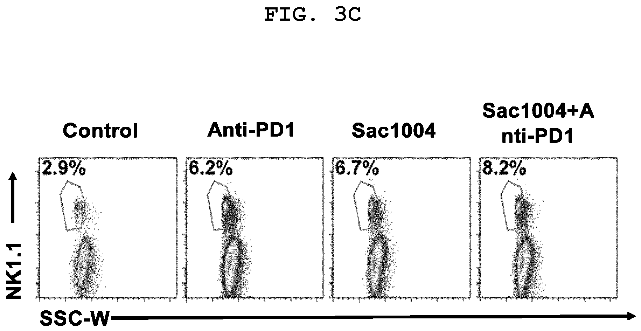

Figures 3a to 3c are the results of FACS of the tumor of the mouse injected with MC38 colorectal cancer cell line and administered with drugs.

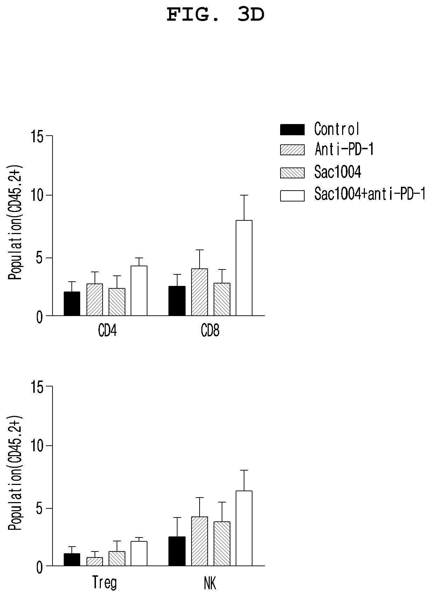

Figure 3d is a graph showing the level of immune factors in each drug administration group through the CD45.2+ marker.

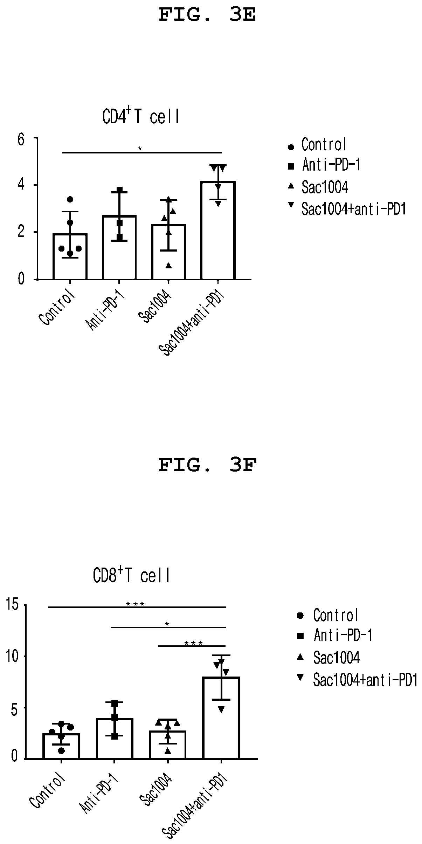

Figure 3e is a graph showing the level of CD4⁺T cells in each drug administration group.

Figure 3f is a graph showing the level of CD8⁺T cells in each drug administration group.

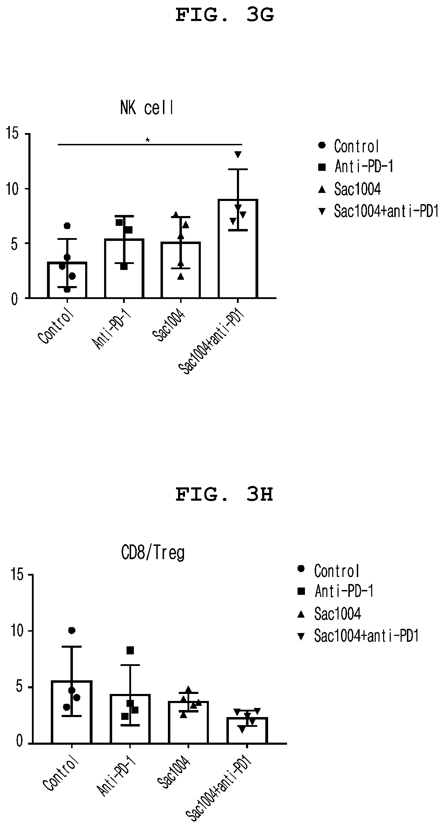

Figure 3g is a graph showing the level of natural killer cells in each drug administration group.

Figure 3h is a graph showing the level of regulatory T cells in each drug administration group.

Particularly, in the SAC-1004 and anti-PD1 co-administration group, the levels of cytotoxic T cells as well as helper T cells were significantly high, the level of natural killer cells was also high, but the level of regulatory T cells that suppressed cytotoxic T cells was decreased. The results were statistically proved by one-way ANOVA.

Therefore, it was confirmed that the effect of activating T cells is greater when SAC-1004 and the cancer immunotherapy agent are administered in combination than when administered alone.

The above results indicate that the effect of activating T cells, an immune-related factor, was superior when SAC-1004 and the cancer immunotherapy agent were administered in combination than when administered alone. From the above results, it was confirmed that the pharmaceutical composition for use in co-administration of the present invention can enhance the anticancer effect of the cancer immunotherapy agent by activating immune factors.

### Experimental Example 7: Analysis of proliferative capacity of immune factors in mouse tumor

In a total of 4 groups consisting of control group, SAC-1004 and anti-PD1 co-administration group, SAC-1004 single administration group, and anti-PD1 single administration group, the proliferative capacity of immune factors in the tumors of mice according to the drug administration for 7 days as described above in the experimental animal model was analyzed. To measure the proliferative capacity, Ki67, a proliferation marker, was used.

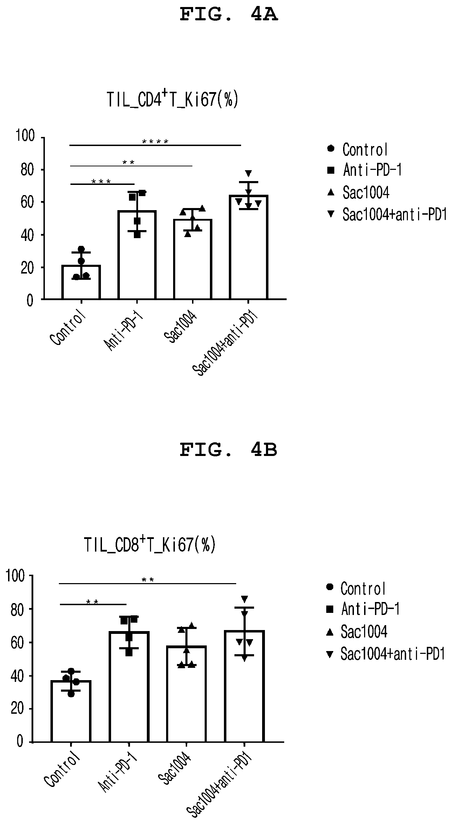

Figure 4a is a graph showing the proliferative capacity of CD4⁺T cells in each drug administration group as total%.

Figure 4b is a graph showing the proliferative capacity of CD8⁺T cells in each drug administration group as total%.

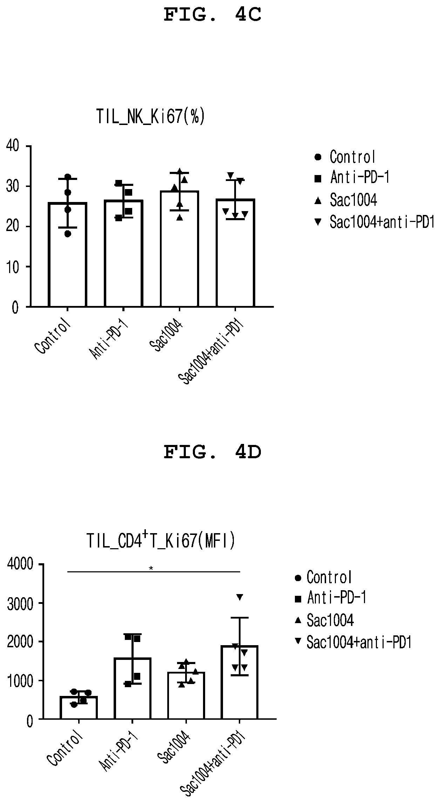

Figure 4c is a graph showing the proliferative capacity of natural miller cells in each drug administration group as total%.

Figure 4d is a graph showing the proliferative capacity of CD4⁺T cells in each drug administration group as MFI (mean fluorescence intensity).

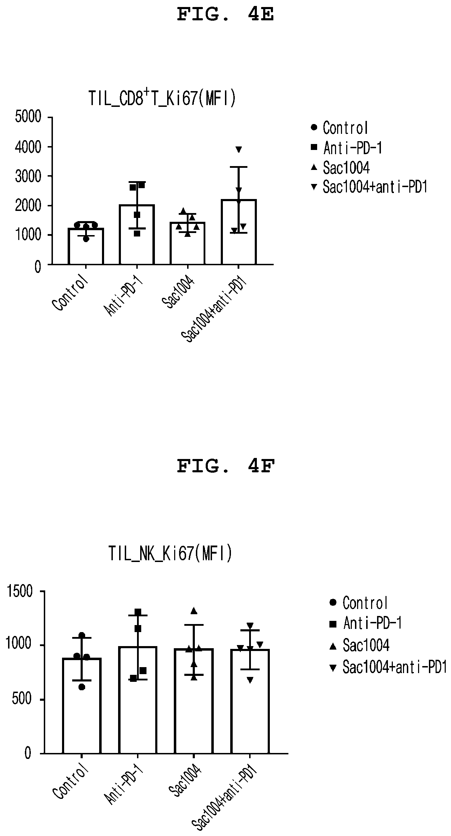

Figure 4e is a graph showing the proliferative capacity of CD8⁺T cells in each drug administration group as MFI (mean fluorescence intensity).

Figure 4f is a graph showing the proliferative capacity of natural killer cells in each drug administration group as MFI (mean fluorescence intensity).

Particularly, the proliferative capacity of helper T cells, cytotoxic T cells, and natural killer cells was higher in the SAC-1004 and anti-PD1 co-administration group than in the other groups.

The above results indicate that when SAC-1004 and the cancer immunotherapy agent were administered in combination, the effect of enhancing the proliferative capacity of the immune-related factors was superior to when SAC-1004 and the cancer immunotherapy agent were administered alone. From the above results, it was confirmed that the pharmaceutical composition for use in co-administration of the present invention can enhance the anticancer effect of the cancer immunotherapy agent by activating immune factors.

### Experimental Example 8: Analysis of T cell cytotoxicity in mouse tumor

In a total of 4 groups consisting of control group, SAC-1004 and anti-PD1 co-administration group, SAC-1004 single administration group, and anti-PD1 single administration group, the T cell cytotoxicity in the tumors of mice according to the drug administration for 7 days as described above in the experimental animal model was analyzed.

To measure the cytotoxicity, CD107a, which is involved in the degranulation of natural killer cells and the activation of CD8⁺T cells, was used as a marker, and the peptide and PMA/ionomycin were treated to activate T cells. In the group treated with the peptide, the peptide expressed in the tumor was added *in vitro.* In the group treated with PMA/ionomycin, all T cells were activated by stimulating the T cell calcium signal.

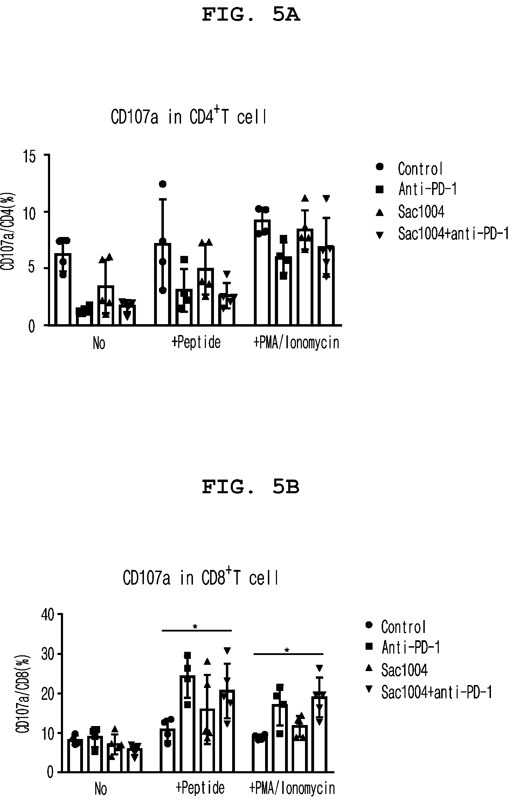

Figure 5a is a graph showing the level of CD107a in CD4⁺T cells in each drug administration group.

Figure 5b is a graph showing the level of CD107a in CD8⁺T cells in each drug administration group.

Particularly, in the group not treated with the peptide and PMA/ionomycin, the level of CD107a in T cells was lower, and the level of CD107a in T cells in the group treated with PMA/ionomycin was higher than that in the group treated with the peptide. In particular, when PMA/ionomycin was treated, the level of CD107a in CD8⁺T cells was high in the SAC-1004 and anti-PD1 co-administration group.

The above results, although relatively increased activation in CD8 + T cells, were not significant in all groups. The cytotoxic effect of activating T cells to kill cancer cells was superior when SAC-1004 and the cancer immunotherapy agent were administered in combination to when administered alone. From the above results, it was confirmed that the pharmaceutical composition for use in co-administration of the present invention can enhance the anticancer effect by co-administration with the cancer immunotherapy agent.

### Experimental Example 9: Analysis of cytokine production capacity of T cells in mouse tumor

In a total of 4 groups consisting of control group, SAC-1004 and anti-PD1 co-administration group, SAC-1004 single administration group, and anti-PD1 single administration group, the cytokine production capacity of T cells in the tumors of mice according to the drug administration for 7 days as described above in the experimental animal model was analyzed.

(9-1)
In order to measure the cytokine production capacity, the level of pro-inflammatory cytokines can be compared, and TNFα, a pro-inflammatory cytokine marker, can be used. As in Experimental Example 8, the level of TNFα was measured after treatment with the peptide and PMA/ionomycin to activate T cells.

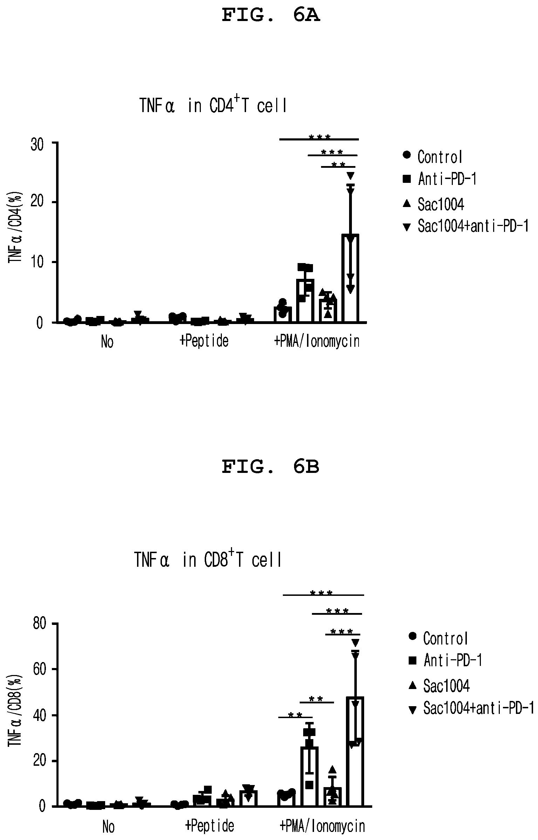

Figure 6a is a graph showing the level of TNFα in CD4⁺T cells in each drug administration group.

Figure 6b is a graph showing the level of TNFα in CD8⁺T cells in each drug administration group.

Particularly, when the peptide and PMA/ionomycin were not treated, the difference in the level of TNFα was not significant in each drug administration group, but when the peptide and PMA/ionomycin were treated, the level of TNFα was significantly increased in SAC-1004 and anti-PD1 co-administration group.

The above results indicate that the effect of activating T cells, an immune-related factor, was superior when SAC-1004 and the cancer immunotherapy agent were administered in combination rather than when administered alone. From the above results, it was confirmed that the pharmaceutical composition for use in co-administration of the present invention can enhance the anticancer effect of the cancer immunotherapy agent by activating immune factors.

(9-2)
IFNγ is also a proinflammatory cytokine marker. Similarly, the level of IFNγ was measured after treatment with the peptide and PMA/ionomycin to activate T cells.

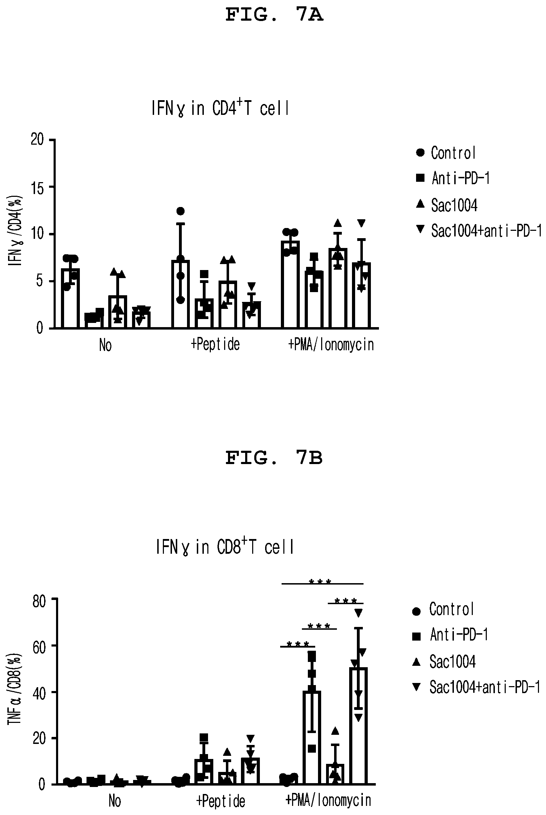

Figure 7a is a graph showing the level of IFNγ in CD4⁺T cells in each drug administration group.

Figure 7b is a graph showing the level of IFNγ in CD8⁺T cells in each drug administration group.

Particularly, when the peptide and PMA/ionomycin were not treated, the difference in the level of IFNγ was not significant in each drug administration group, but when the peptide and PMA/ionomycin were treated, the level of IFNγ was significantly increased in SAC-1004 and anti-PD1 co-administration group.

The above results indicate that the effect of activating T cells, an immune-related factor, was superior when SAC-1004 and the cancer immunotherapy agent were administered in combination rather than when administered alone. From the above results, it was confirmed that the pharmaceutical composition for use in co-administration of the present invention can enhance the anticancer effect of the cancer immunotherapy agent by activating immune factors.

### Experimental Example 10: Analysis of cytokine production capacity of T cells in mouse spleen

In a total of 4 groups consisting of control group, SAC-1004 and anti-PD1 co-administration group, SAC-1004 single administration group, and anti-PD1 single administration group, FACS analysis, cytotoxicity analysis, and cytokine production capacity analysis were performed using CD107a, TNFα and IFNγ markers to compare the functions of T cells in the spleen of mice treated with the drug for 7 days as described above in the experimental animal model. In addition, as in Experimental Examples 8 and 9, the peptide and PMA/ionomycin were treated to activate T cells.

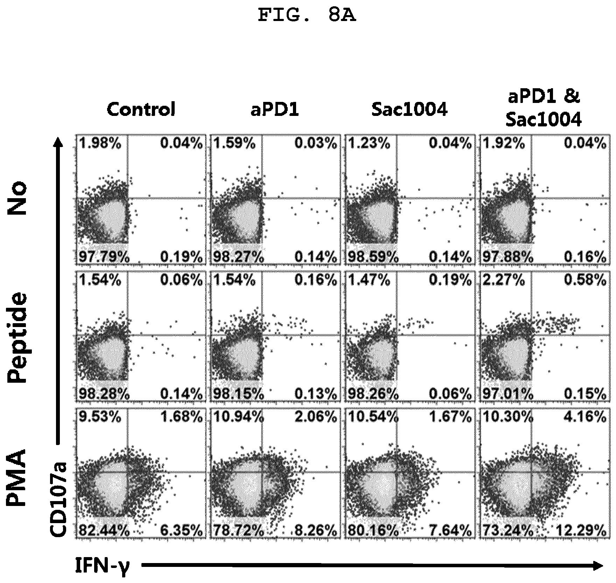

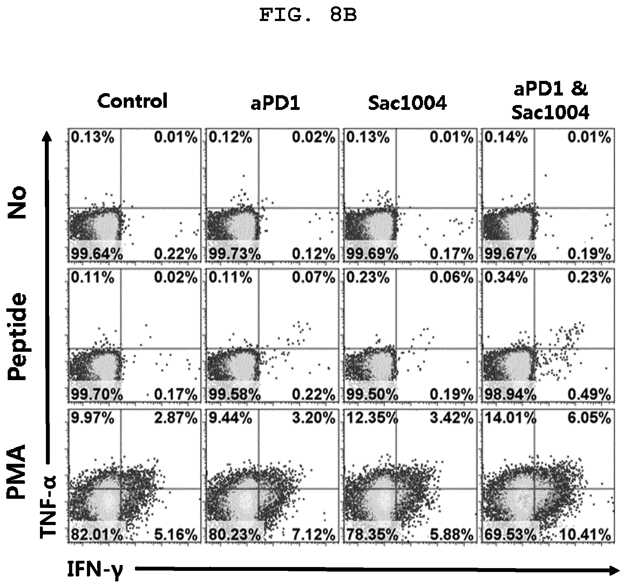

Figures 8a and 8b are the results of FACS of the tumor of the mouse injected with MC38 colorectal cancer cell line and administered with drugs.

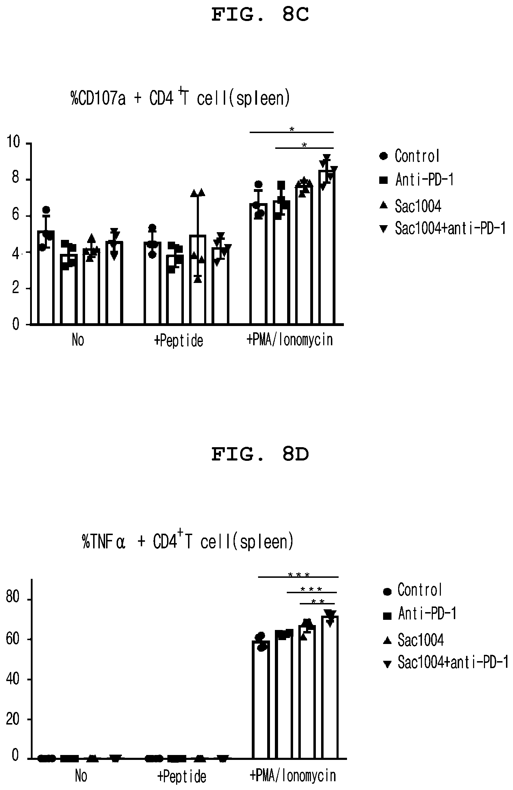

Figure 8c is a graph showing the CD107a level of CD4⁺T cells in the spleen of each drug administration group.

Figure 8d is a graph showing the TNFα level of CD4⁺T cells in the spleen of each drug administration group.

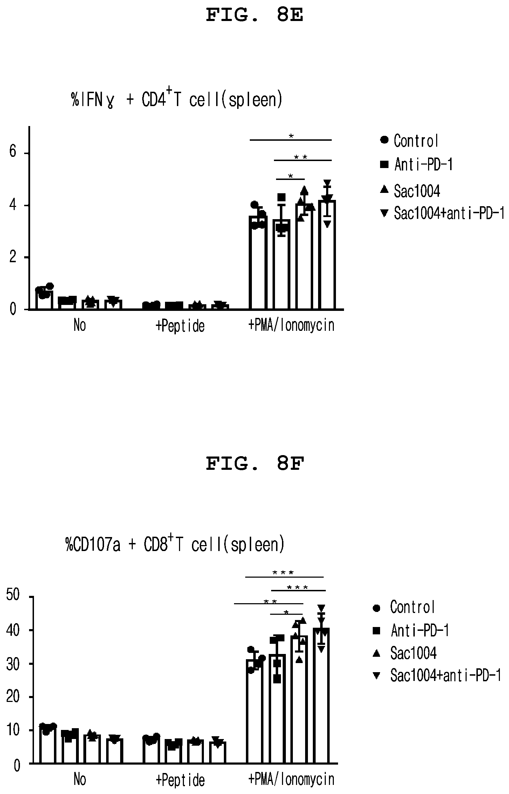

Figure 8e is a graph showing the IFNγ level of CD4⁺T cells in the spleen of each drug administration group.

Figure 8f is a graph showing the CD107a level of CD8⁺T cells in the spleen of each drug administration group.

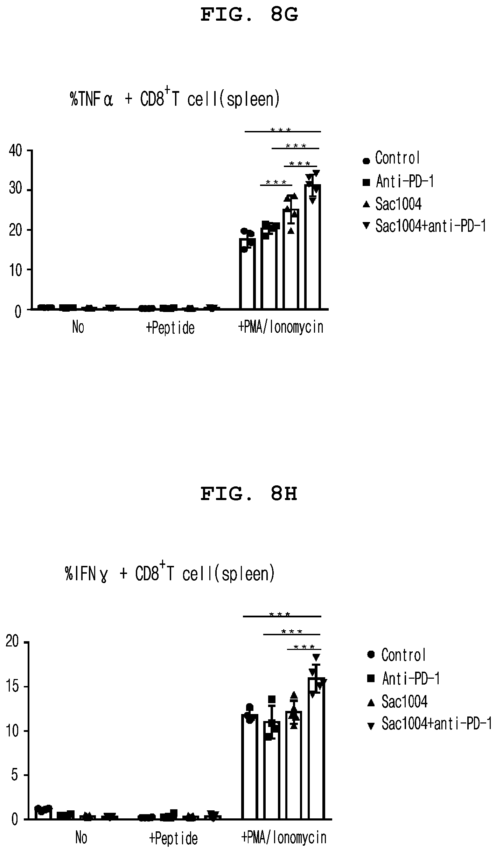

Figure 8g is a graph showing the TNFα level of CD8⁺T cells in the spleen of each drug administration group.

Figure 8h is a graph showing the IFNγ level of CD8⁺T cells in the spleen of each drug administration group.

Particularly, when T cells were activated by treating CD4⁺T cells and CD8⁺T cells in the spleen with PMA/ionomycin, the levels of CD107a, TNFα and IFNγ were increased in the SAC-1004 and anti-PD1 co-administration group.

The above results indicate that the cytotoxic effect of killing cancer cells was superior and the effect of activating T cells, an immune-related factor, was also superior when SAC-1004 and the cancer immunotherapy agent were administered in combination rather than when administered alone. From the above results, it was confirmed that the pharmaceutical composition for use in co-administration of the present invention can enhance the anticancer effect of the cancer immunotherapy agent by activating immune factors.

### Experimental Example 11: Analysis of adherent junction expression in mouse tumor

In a total of 4 groups consisting of control group, SAC-1004 and anti-PD1 co-administration group, SAC-1004 single administration group, and anti-PD1 single administration group, the expression of adherent junction in the tumor of the mouse treated with the drug as described above in the experimental animal model was analyzed except that the drug was administered for 14 days by daily administration of SAC-1004 for 7 days and administration of anti-PD1 3 times a week.

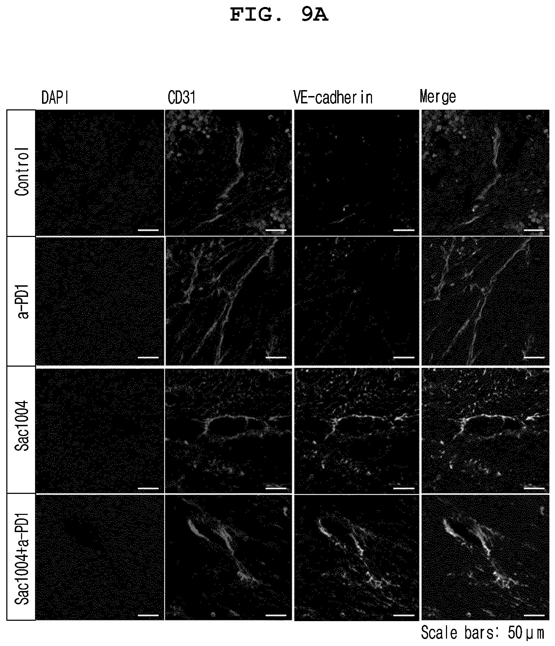

Figure 9a is a fluorescence photograph showing the expression level of intratumoral adherent junction in each drug administration group.

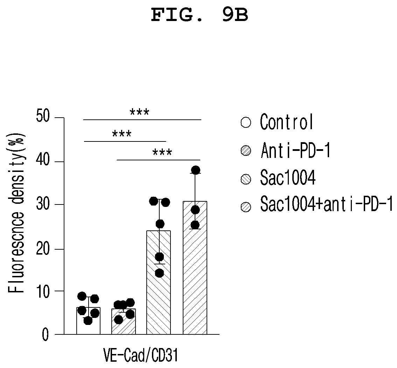

Figure 9b is a graph showing the fluorescence density by quantifying the expression level of intratumoral adherent junction in each drug administration group.

Particularly, Figure 9a shows the results of IHC staining, DAPI was used to stain the nucleus, CD31 was used to stain blood vessels, and VE-cadherin (adhesion protein) was used to view adherent junction. At this time, if VE-cadherin is well expressed, drug delivery is good and the anticancer effect is increased, and the adhesion between endothelial cells is good, blood vessels are stabilized, and T cells are well infiltrated into the tumor.

As shown in Figures 9a and 9b, the ratio of intratumoral VE-cadherin was increased in the SAC-1004 single administration group than that in the control group and the anti-PD1 single administration group, and intratumoral VE-cadherin was more expressed in the SAC-1004 and anti-PD1 co-administration group than in the SAC-1004 single administration group.

The above results indicate that the cancer immunotherapy agent was well delivered, T cells penetrated well into the tumor, and the anticancer effect was increased when SAC-1004 and the cancer immunotherapy agent were administered in combination rather than when administered alone. From the above results, it was confirmed that the pharmaceutical composition for use in co-administration of the present invention can enhance the anticancer effect of the cancer immunotherapy agent.

### Experimental Example 12: Analysis of PDL1 expression in mouse tumor

In a total of 4 groups consisting of control group, SAC-1004 and anti-PD1 co-administration group, SAC-1004 single administration group, and anti-PD1 single administration group, the expression of PDL1 (programmed death-ligand 1) in the tumors of mice according to the drug administration for 7 days as described above in the experimental animal model was analyzed. PDL1 is a ligand expressed in tumors, and CD3 is used as a T cell marker.

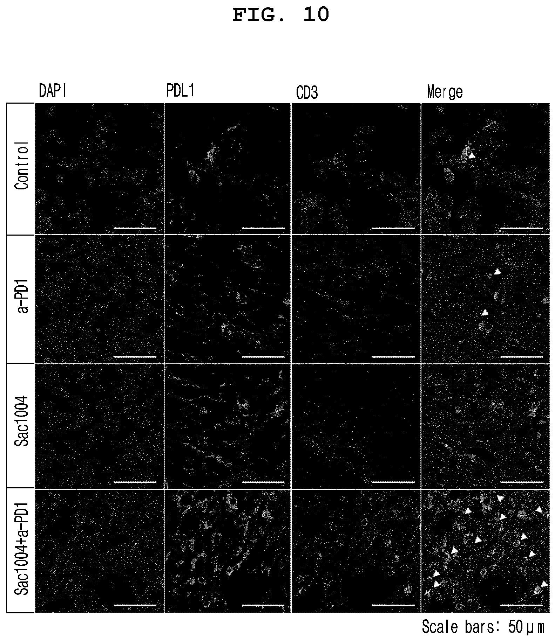

Figure 10a is a fluorescence photograph showing the expression levels of PDL1 and CD3 in each drug administration group.

Particularly, Figure 10a shows the results of IHC staining, and the increase in PDL1 means that anti-PD1 was well delivered and immune activation occurred. The expression levels of PDL1 and CD3 in the SAC-1004 and anti-PD1 co-administration group were higher than those in the anti-PD1 single administration group.

The above results indicate that the cancer immunotherapy agent was well delivered, immune activation occurred well, and the anticancer effect was increased when SAC-1004 and the cancer immunotherapy agent were administered in combination rather than when administered alone. From the above results, it was confirmed that the pharmaceutical composition for use in co-administration of the present invention can enhance the anticancer effect of the cancer immunotherapy agent.

### Experimental Example 13: Analysis of cytokine expression level in mouse tumor

In a total of 4 groups consisting of control group, SAC-1004 and anti-PD1 co-administration group, SAC-1004 single administration group, and anti-PD1 single administration group, the expression level of cytokines in the tumors of mice according to the drug administration for 7 days as described above in the experimental animal model was analyzed. As cytokine markers, pro-inflammatory cytokines and anti-inflammatory cytokines were used, and RT-PCR was performed.

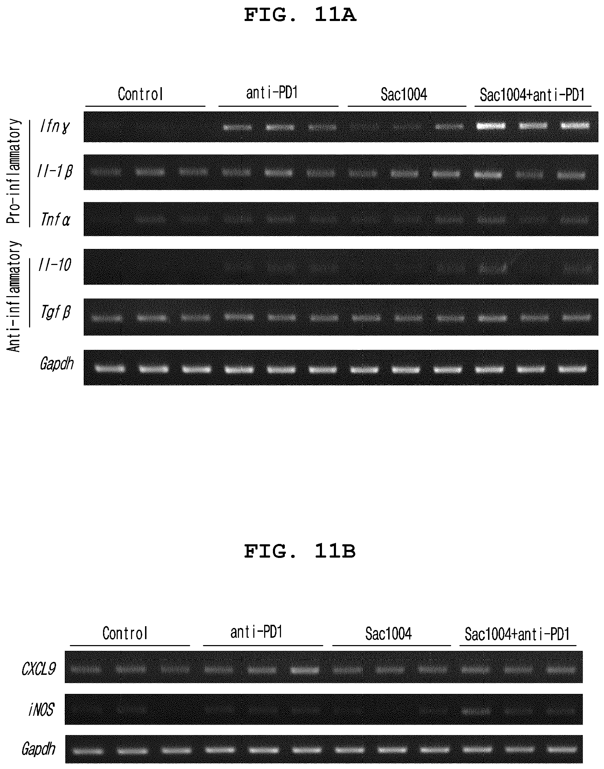

Figure 11a is a photograph showing the results of RT-PCR for the expressions of pro-inflammatory cytokines and anti-inflammatory cytokines in each drug administration group.

Figure 11b is a photograph showing the results of RT-PCR for the expressions of CXCL9, iNOS and Gapdh in each drug administration group.

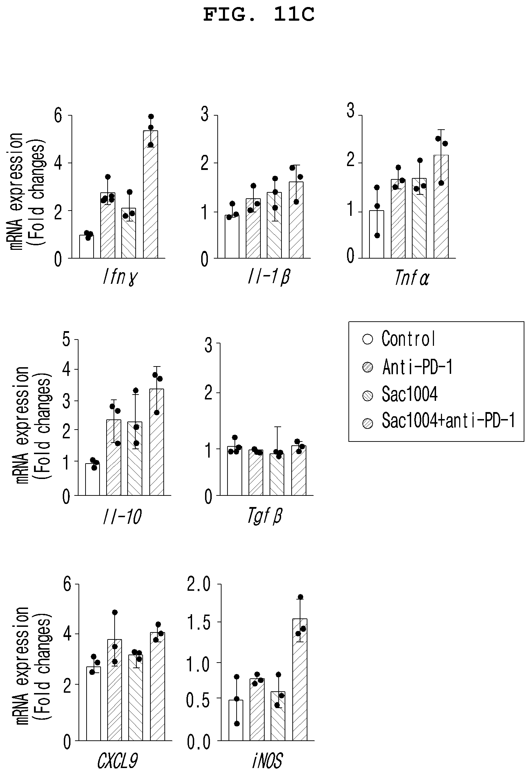

Figure 11c is a photograph showing the mRNA expression level graphically through RT-PCR for each group.

Particularly, among pro-inflammatory cytokines, IFNγ was increased in the group administered with anti-PD1, and it was more increased in the SAC-1004 and anti-PD1 co-administration group than in the group administered with anti-PD1.

The above results indicate that the effect of activating T cells, an immune-related factor, was superior when SAC-1004 and the cancer immunotherapy agent were administered in combination rather than when administered alone. From the above results, it was confirmed that the pharmaceutical composition for use in co-administration of the present invention can enhance the anticancer effect of the cancer immunotherapy agent by activating immune factors.

### Experimental Example 14: Analysis of survival rate of mice according to drug administration after depletion of CD4/8⁺T and NK cells at the time of growth of MC38 colorectal cancer cell line and drug injection

In a total of 4 groups consisting of control group, SAC-1004 and anti-PD1 co-administration group, SAC-1004 single administration group, and anti-PD1 single administration group, the survival rate of mice according to the drug administration for 7 days as described above in the experimental animal model after depletion of CD4/8⁺T and NK cells was analyzed.

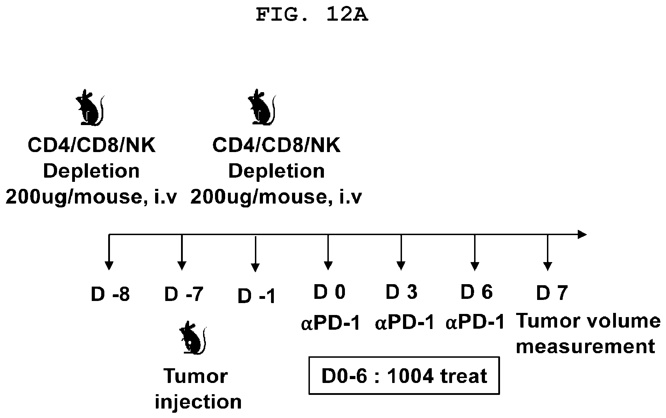

Figure 12a is a schematic diagram showing the procedure according to CD4/8+T, NK removal, MC38 colorectal cancer cell line injection and drug administration.

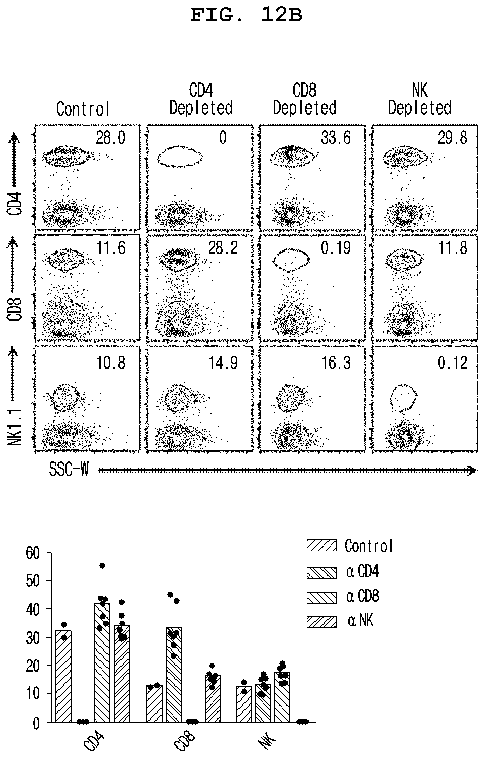

Figure 12b is a diagram confirming that the results according to CD4/8+T, NK removal, MC38 colorectal cancer cell line injection and drug administration were confirmed through flow cytometry, and that the removal of immune cells proceeded smoothly.

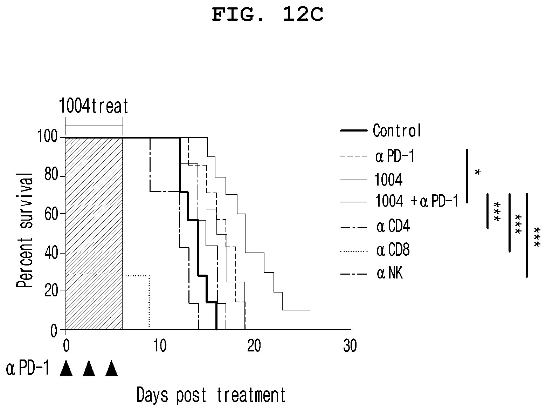

Figure 12c is a diagram showing the survival rate of the mouse according to CD4/8+T and NK removal after the MC38 colorectal cancer cell line injection and drug administration, and confirming that the experimental group mouse in which CD8+ T cells were removed had the lowest survival rate.

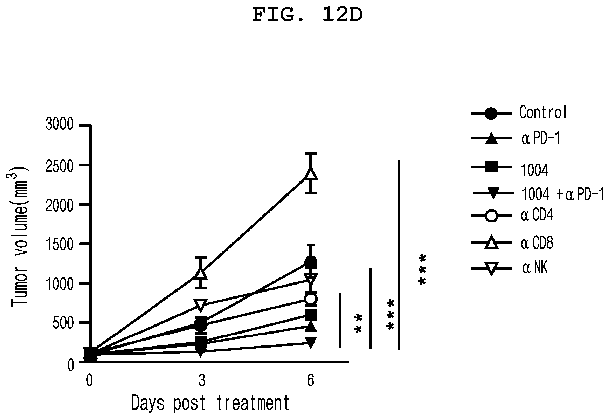

Figure 12d is a diagram illustrating the same process as Figure 12c, showing the growth rate of the tumor according to the CD4/8+T removal over time after the MC38 colorectal cancer cell line injection and drug administration, and confirming that the tumor growth rate was the highest in the experimental group mouse in which CD8+ T cells were removed.

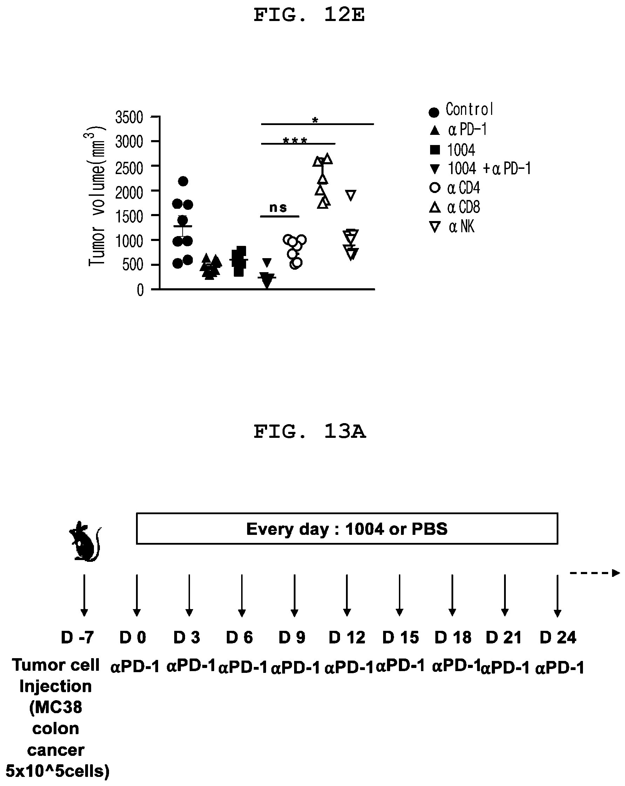

Figure 12e is a diagram showing the comparison of the tumor size according to the CD4/8+T and NK removal in each experimental group after the MC38 colorectal cancer cell line injection and drug administration, and confirming that the tumor size was the largest in the experimental group mouse in which CD8+ T cells were removed.

The above results indicate that the removal of CD8⁺T cells showed the greatest tumor growth rate, and that the experimental group in which NK cells and CD4⁺T cells were removed showed a great tumor growth rate. This suggests that the co-administration of SAC-1004 and anti-PD1 is a CD8⁺T cell-dependently induced process.

### Experimental Example 15: Analysis of results according to long-term drug administration

In a total of 4 groups consisting of control group, SAC-1004 and anti-PD1 co-administration group, SAC-1004 single administration group, and anti-PD1 single administration group, the survival rate was analyzed after long-term drug injection.

Figure 13a is a schematic diagram showing the procedure of the MC38 colorectal cancer cell line injection and long-term drug administration.

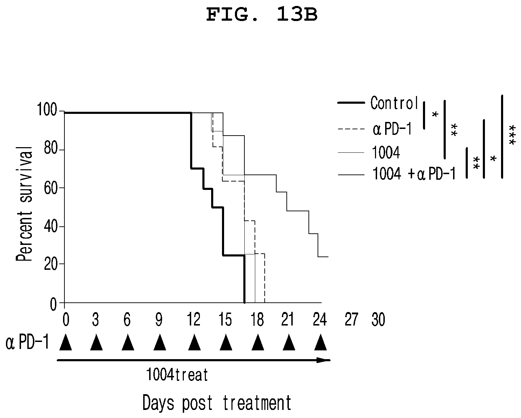

Figure 13b is a graph showing the survival rate of the mouse according to the MC38 colorectal cancer cell line injection and long-term drug administration.

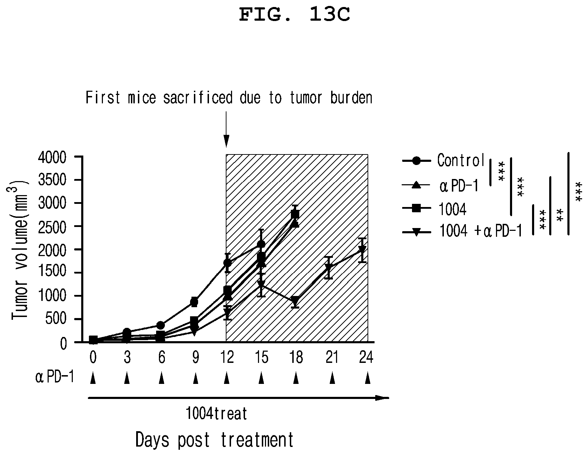

Figure 13c is a graph showing the tumor size in the mouse according to the MC38 colorectal cancer cell line injection and long-term drug administration.

The same results were consistently shown even after long-term drug treatment. Therefore, the reliability of the above experimental procedure was confirmed, and it was proved that there was no drug toxicity despite long-term drug administration.

### Manufacturing Example 1: Preparation of powders

| | |
|---|---|
| Compound represented by formula 1 | 2 g |
| Lactose | 1 g |

Powders were prepared by mixing all the above components, which were filled in airtight packs according to the conventional method for preparing powders.

### Manufacturing Example 2: Preparation of tablets

| | |
|---|---|
| Compound represented by formula 1 | 100 mg |
| Corn starch | 100 mg |
| Lactose | 100 mg |
| Magnesium stearate | 2 mg |

Tablets were prepared by mixing all the above components by the conventional method for preparing tablets.

### Manufacturing Example 3: Preparation of capsules

| | |
|---|---|
| Compound represented by formula 1 | 100 mg |
| Corn starch | 100 mg |
| Lactose | 100 mg |
| Magnesium stearate | 2 mg |

Capsules were prepared by mixing all the above components, which were filled in gelatin capsules according to the conventional method for preparing capsules.

### Manufacturing Example 4: Preparation of injectable solutions

| | |
|---|---|
| Compound represented by formula 1 | 100 mg |
| Mannitol | 180 mg |
| Na₂HPO₄ • 2H₂O | 26 mg |
| Distilled water | 2974 mg |

Injectable solutions were prepared by mixing all the above components by the conventional method for preparing injectable solutions.

### Manufacturing Example 5: Preparation of ointments

| | |
|---|---|
| Compound represented by formula 1 | 5 g |
| Cetyl palmitate | 20 g |
| Cetanol | 40 g |
| Stearyl alcohol | 40 g |
| Isopropyl myristate | 80 g |
| Polysorbate | 60 g |
| Propyl p-hydroxybenzoate | 1 g |
| Methyl p-hydroxybenzoate | 1 g |

Phosphoric acid and purified water proper amount

Ointments were prepared by mixing all the above components by the conventional method for preparing ointments.

## Claims

1. Compound represented by formula 2 below, a stereoisomer thereof, or a pharmaceutically acceptable salt thereof, for use in enhancing immunity in cancer:

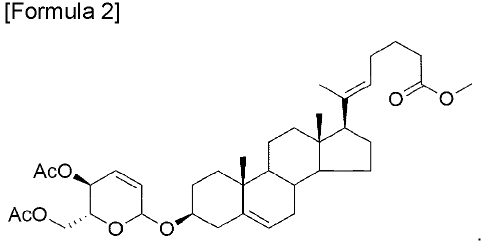

2. Compound for use according to claim 1, wherein the cancer is one or more selected from the group consisting of pseudomyxoma, intrahepatic cholangiocarcinoma, hepatoblastoma, liver cancer, thyroid cancer, colon cancer, testicular cancer, myelodysplastic syndrome, glioblastoma, oral cancer, lip cancer, mycosis fungoides, acute myeloid leukemia, acute lymphoblastic leukemia, basal cell carcinoma, ovarian epithelial cancer, ovarian germ cell cancer, male breast cancer, brain cancer, pituitary adenoma, multiple myeloma, gallbladder cancer, biliary tract cancer, colorectal cancer, chronic myelogenous leukemia, chronic lymphocytic leukemia, retinoblastoma, choroidal melanoma, ampullar of vater cancer, bladder cancer, peritoneal cancer, parathyroid cancer, adrenal cancer, nasal cavity cancer, non-small cell lung cancer, tongue cancer, astrocytoma, small cell lung cancer, juvenile brain cancer, juvenile lymphoma, juvenile leukemia, small intestine cancer, meningioma, esophageal cancer, glioma, renal pelvic cancer, kidney cancer, heart cancer, duodenal cancer, malignant soft tissue cancer, malignant bone cancer, malignant lymphoma, malignant mesothelioma, malignant melanoma, eye cancer, vulvar cancer, ureter cancer, urethral cancer, cancer of unknown primary site, gastric lymphoma, stomach cancer, gastric carcinoma, gastrointestinal stromal cancer, Wilms cancer, breast cancer, triple-negative breast cancer, sarcoma, penile cancer, pharyngeal cancer, gestational villous disease, cervical cancer, endometrial cancer, uterine sarcoma, prostate cancer, metastatic bone cancer, metastatic brain cancer, mediastinal cancer, rectal cancer, rectal carcinoma, vaginal cancer, spinal cord cancer, vestibular schwannoma, pancreatic cancer, salivary gland cancer, Kaposi's sarcoma, Paget's disease, tonsil cancer, squamous cell carcinoma, lung adenocarcinoma, lung cancer, lung squamous cell carcinoma, skin cancer, anal cancer, rhabdomyosarcoma, laryngeal cancer, pleural cancer, blood cancer and thymus cancer.

3. Compound for use according to claim 2, wherein the cancer is colon cancer.

4. Compound represented by formula 2 below, a stereoisomer thereof, or a pharmaceutically acceptable salt thereof, for use in enhancing the efficacy of a cancer immunotherapy agent, wherein the cancer immunotherapy agent is anti-PD1:

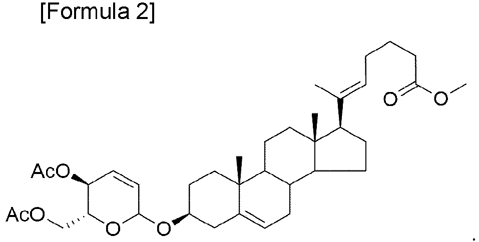

5. Combination drug comprising:
a cancer immunotherapy agent, wherein the cancer immunotherapy agent is anti-PD1; and
a compound represented by formula 2 below, a stereoisomer thereof, or a pharmaceutically acceptable salt thereof:

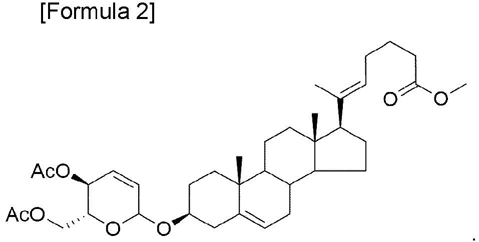

6. Combination drug for use in cancer immunotherapy treatment, wherein the combination drug is defined according to claim 5.

7. Combination drug for use according to claim 6, wherein the compound, the stereoisomer thereof, or the pharmaceutically acceptable salt thereof is administered simultaneously with or sequentially to the anti-PD1.

8. Combination drug for use according to any one of claims 6 or 7, wherein the use is in immunotherapy treatment of at least one cancer selected from the group consisting of pseudomyxoma, intrahepatic cholangiocarcinoma, hepatoblastoma, liver cancer, thyroid cancer, colon cancer, testicular cancer, myelodysplastic syndrome, glioblastoma, oral cancer, lip cancer, mycosis fungoides, acute myeloid leukemia, acute lymphoblastic leukemia, basal cell carcinoma, ovarian epithelial cancer, ovarian germ cell cancer, male breast cancer, brain cancer, pituitary adenoma, multiple myeloma, gallbladder cancer, biliary tract cancer, colorectal cancer, chronic myelogenous leukemia, chronic lymphocytic leukemia, retinoblastoma, choroidal melanoma, ampullar of vater cancer, bladder cancer, peritoneal cancer, parathyroid cancer, adrenal cancer, nasal cavity cancer, non-small cell lung cancer, tongue cancer, astrocytoma, small cell lung cancer, juvenile brain cancer, juvenile lymphoma, juvenile leukemia, small intestine cancer, meningioma, esophageal cancer, glioma, renal pelvic cancer, kidney cancer, heart cancer, duodenal cancer, malignant soft tissue cancer, malignant bone cancer, malignant lymphoma, malignant mesothelioma, malignant melanoma, eye cancer, vulvar cancer, ureter cancer, urethral cancer, cancer of unknown primary site, gastric lymphoma, stomach cancer, gastric carcinoma, gastrointestinal stromal cancer, Wilms cancer, breast cancer, triple-negative breast cancer, sarcoma, penile cancer, pharyngeal cancer, gestational villous disease, cervical cancer, endometrial cancer, uterine sarcoma, prostate cancer, metastatic bone cancer, metastatic brain cancer, mediastinal cancer, rectal cancer, rectal carcinoma, vaginal cancer, spinal cord cancer, vestibular schwannoma, pancreatic cancer, salivary gland cancer, Kaposi's sarcoma, Paget's disease, tonsil cancer, squamous cell carcinoma, lung adenocarcinoma, lung cancer, lung squamous cell carcinoma, skin cancer, anal cancer, rhabdomyosarcoma, laryngeal cancer, pleural cancer, blood cancer and thymus cancer.

9. Combination drug for use according to claim 8, wherein the cancer is colon cancer.

10. A compound, a stereoisomer thereof, or a pharmaceutically acceptable salt thereof for use according to any one of claims 1-4, combination drug for use according to any one of claims 6-9, wherein the compound, the stereoisomer thereof, or the pharmaceutically acceptable salt thereof activates at least one immune factor selected from the group consisting of helper T cells, cytotoxic T cells, natural killer cells (NK cells) and cytokines.

11. Kit comprising a cancer immunotherapy agent and a compound represented by formula 2 below, a stereoisomer thereof, or a pharmaceutically acceptable salt thereof,
wherein the cancer immunotherapy agent is anti-PD1:

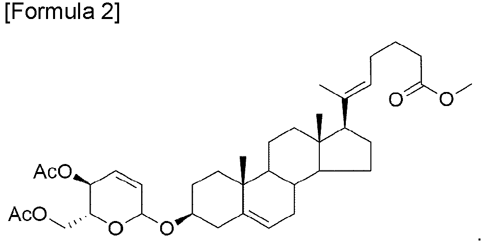

## Patentansprüche

1. Verbindung, die durch nachstehende Formel 2 wiedergegeben wird, Stereoisomer davon oder pharmazeutisch unbedenkliches Salz davon zur Verwendung bei der Erhöhung der Immunität bei Krebs:

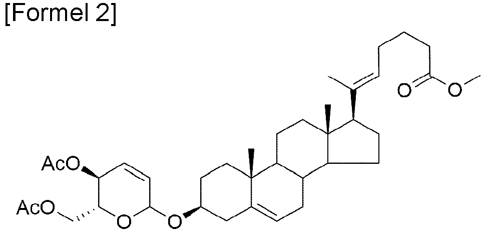

2. Verbindung zur Verwendung nach Anspruch 1, wobei es sich bei dem Krebs um einen oder mehrere aus der Gruppe bestehend aus Pseudomyxom, intrahepatischem Cholangiokarzinom, Hepatoblastom, Leberkrebs, Schilddrüsenkrebs, Kolonkrebs, Hodenkrebs, myelodysplastischem Syndrom, Glioblastom, Mundkrebs, Lippenkrebs, Mycosis fungoides, akuter myeloischer Leukämie, akuter lymphoblastischer Leukämie, Basalzellenkrebs, Eierstockepithelkarzinom, Eierstock-Keimzellenkarzinom, männlichem Brustkrebs, Gehirnkrebs, Hypophysenadenom, multiplem Myelom, Gallenblasenkrebs, Gallengangkrebs, Kolorektalkrebs, chronischer myeloischer Leukämie, chronischer lymphatischer Leukämie, Retinoblastom, Aderhautmelanom, Vater-Ampullen-Krebs, Blasenkrebs, Peritonealkrebs, Nebenschilddrüsenkrebs, Nebennierenkrebs, Nasenhöhlenkrebs, nichtkleinzelligem Lungenkrebs, Zungenkrebs, Astrozytom, kleinzelligem Lungenkrebs, juvenilen Hirnkrebs, juveniles Lymphom, juvenile Leukämie, Dünndarmkrebs, Meningiom, Speiseröhrenkrebs, Gliom, Nierenbeckenkrebs, Nierenkrebs, Herzkrebs, Zwölffingerdarmkrebs, malignem Weichteilkrebs, malignem Knochenkrebs, malignem Lymphom, malignem Mesotheliom, malignem Melanom, Augenkrebs, Vulvakrebs, Harnleiterkrebs, Harnröhrenkrebs, Krebs mit unbekanntem Primärort, Lymphom des Magens, Magenkrebs, Magenkarzinom, gastrointestinalem Stromakrebs, Wilms-Krebs, Brustkrebs, dreifach negativem Brustkrebs, Sarkom, Peniskrebs, Rachenkrebs, Schwangerschafts-Zottenerkrankung, Zervixkrebs, Endometriumkrebs, Gebärmuttersarkom, Prostatakrebs, metastatischem Knochenkrebs, metastatischem Gehirnkrebs, Mediastinalkrebs, Rektalkrebs, Rektalkarzinom, Vaginalkrebs, Rückenmarkskrebs, vestibulärem Schwannom, Bauchspeicheldrüsenkrebs, Speicheldrüsenkrebs, Kaposi-Sarkom, Morbus Paget, Mandelkrebs, Plattenepithelkarzinom, Lungenadenokarzinom, Lungenkrebs, Lungenplattenepithelkarzinom, Hautkrebs, Analkrebs, Rhabdomyosarkom, Kehlkopfkrebs, Brustfellkrebs, Blutkrebs und Thymuskrebs handelt.

3. Verbindung zur Verwendung nach Anspruch 2, wobei es sich bei dem Krebs um Kolonkrebs handelt.

4. Verbindung, die durch nachstehende Formel 2 wiedergegeben wird, Stereoisomer davon oder pharmazeutisch unbedenkliches Salz davon zur Verwendung bei der Erhöhung der Wirksamkeit eines Krebsimmuntherapiemittels, wobei es sich bei dem Krebsimmuntherapiemittel um Anti-PD1 handelt:

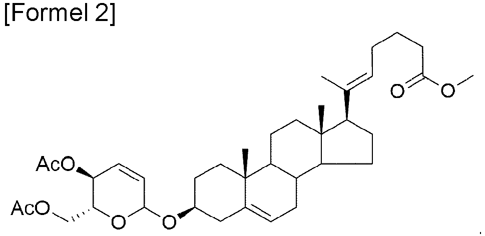

5. Kombinationsarzneimittel, umfassend:
ein Krebsimmuntherapiemittel, wobei es sich bei dem Krebsimmuntherapiemittel um Anti-PD1 handelt; und
eine Verbindung, die durch nachstehende Formel 2 wiedergegeben wird, ein Stereoisomer davon oder ein pharmazeutisch unbedenkliches Salz davon:

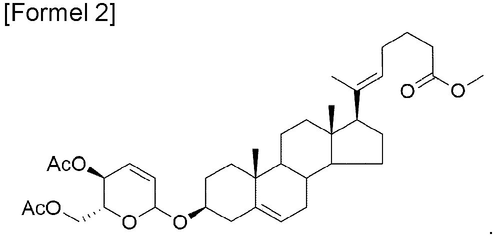

6. Kombinationsarzneimittel zur Verwendung bei der Krebsimmuntherapiebehandlung, wobei das Kombinationsarzneimittel gemäß Anspruch 5 definiert ist.

7. Kombinationsarzneimittel zur Verwendung nach Anspruch 6, wobei die Verbindung, das Stereoisomer davon oder das pharmazeutisch unbedenkliche Salz davon gleichzeitig oder zeitversetzt mit dem Anti-PD1 verabreicht wird.

8. Kombinationsarzneimittel zur Verwendung nach einem der Ansprüche 6 oder 7, wobei die Verwendung bei der Immuntherapie von mindestens einem Krebs aus der Gruppe bestehend aus Pseudomyxom, intrahepatischem Cholangiokarzinom, Hepatoblastom, Leberkrebs, Schilddrüsenkrebs, Kolonkrebs, Hodenkrebs, myelodysplastischem Syndrom, Glioblastom, Mundkrebs, Lippenkrebs, Mycosis fungoides, akuter myeloischer Leukämie, akuter lymphoblastischer Leukämie, Basalzellenkrebs, Eierstockepithelkarzinom, Eierstock-Keimzellenkarzinom, männlichem Brustkrebs, Gehirnkrebs, Hypophysenadenom, multiplem Myelom, Gallenblasenkrebs, Gallengangkrebs, Kolorektalkrebs, chronischer myeloischer Leukämie, chronischer lymphatischer Leukämie, Retinoblastom, Aderhautmelanom, Vater-Ampullen-Krebs, Blasenkrebs, Peritonealkrebs, Nebenschilddrüsenkrebs, Nebennierenkrebs, Nasenhöhlenkrebs, nichtkleinzelligem Lungenkrebs, Zungenkrebs, Astrozytom, kleinzelligem Lungenkrebs, juvenilen Hirnkrebs, juveniles Lymphom, juvenile Leukämie, Dünndarmkrebs, Meningiom, Speiseröhrenkrebs, Gliom, Nierenbeckenkrebs, Nierenkrebs, Herzkrebs, Zwölffingerdarmkrebs, malignem Weichteilkrebs, malignem Knochenkrebs, malignem Lymphom, malignem Mesotheliom, malignem Melanom, Augenkrebs, Vulvakrebs, Harnleiterkrebs, Harnröhrenkrebs, Krebs mit unbekanntem Primärort, Lymphom des Magens, Magenkrebs, Magenkarzinom, gastrointestinalem Stromakrebs, Wilms-Krebs, Brustkrebs, dreifach negativem Brustkrebs, Sarkom, Peniskrebs, Rachenkrebs, Schwangerschafts-Zottenerkrankung, Zervixkrebs, Endometriumkrebs, Gebärmuttersarkom, Prostatakrebs, metastatischem Knochenkrebs, metastatischem Gehirnkrebs, Mediastinalkrebs, Rektalkrebs, Rektalkarzinom, Vaginalkrebs, Rückenmarkskrebs, vestibulärem Schwannom, Bauchspeicheldrüsenkrebs, Speicheldrüsenkrebs, Kaposi-Sarkom, Morbus Paget, Mandelkrebs, Plattenepithelkarzinom, Lungenadenokarzinom, Lungenkrebs, Lungenplattenepithelkarzinom, Hautkrebs, Analkrebs, Rhabdomyosarkom, Kehlkopfkrebs, Brustfellkrebs, Blutkrebs und Thymuskrebs erfolgt.

9. Kombination Arzneimittel zur Verwendung nach Anspruch 8, wobei es sich bei dem Krebs um Kolonkrebs handelt.

10. Verbindung, Stereoisomer davon oder pharmazeutisch unbedenkliches Salz davon zur Verwendung nach einem der Ansprüche 1-4, Kombinationsarzneimittel zur Verwendung nach einem der Ansprüche 6-9, wobei die Verbindung, das Stereoisomer davon oder das pharmazeutisch unbedenkliche Salz davon mindestens einen Immunfaktor aktiviert, der aus der Gruppe bestehend aus Helfer-T-Zellen, zytotoxischen T-Zellen, natürlichen Killerzellen (NK-Zellen) und Zytokinen ausgewählt ist.

11. Kit, umfassend ein Krebsimmuntherapiemittel und eine Verbindung, die durch nachstehende Formel 2 wiedergegeben wird, ein Stereoisomer davon oder ein pharmazeutisch unbedenkliches Salz davon,
wobei es sich bei dem Krebsimmuntherapiemittel um Anti-PD1 handelt:

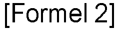

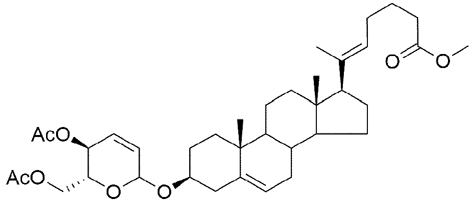

## Revendications

1. Composé représenté par la formule 2 ci-dessous, stéréoisomère correspondant, ou sel pharmaceutiquement acceptable correspondant, pour une utilisation pour améliorer l'immunité dans le cancer :

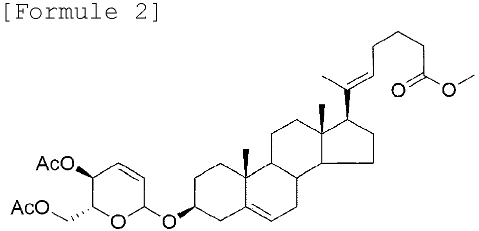

2. Composé pour une utilisation selon la revendication 1, dans lequel le cancer est un ou plusieurs choisis dans le groupe constitué par le pseudomyxome, le cholangiocarcinome intrahépatique, l'hépatoblastome, le cancer du foie, le cancer de la thyroïde, le cancer du côlon, le cancer des testicules, le syndrome myélodysplasique, le glioblastome, le cancer de la bouche, le cancer des lèvres, le mycosis fongoïde, la leucémie myéloïde aiguë, la leucémie aiguë lymphoblastique, le carcinome basocellulaire, le cancer épithélial de l'ovaire, le cancer des cellules germinales de l'ovaire, le cancer du sein masculin, le cancer du cerveau, l'adénome hypophysaire, le myélome multiple, le cancer de la vésicule biliaire, le cancer des voies biliaires, le cancer colorectal, la leucémie myéloïde chronique, la leucémie lymphoïde chronique, le rétinoblastome, le mélanome choroïdien, le cancer de l'ampoule de Vater, le cancer de la vessie, le cancer péritonéal, le cancer de la parathyroïde, le cancer des surrénales, le cancer des fosses nasales, le cancer du poumon non à petites cellules, le cancer de la langue, l'astrocytome, le cancer du poumon à petites cellules, le cancer juvénile du cerveau, le lymphome juvénile, la leucémie juvénile, le cancer de l'intestin grêle, le méningiome, le cancer de l'œsophage, le gliome, le cancer du bassinet, le cancer du rein, le cancer du cœur, le cancer du duodénum, le cancer malin des tissus mous, le cancer malin des os, le lymphome malin, le mésothéliome malin, le mélanome malin, le cancer de l'œil, le cancer de la vulve, le cancer de l'uretère, le cancer de l'urètre, le cancer de site primitif inconnu, le lymphome gastrique, le cancer de l'estomac, le carcinome gastrique, le cancer du stroma gastro-intestinal, le cancer de Wilms, le cancer du sein, le cancer du sein triple négatif, le sarcome, le cancer du pénis, le cancer du pharynx, la maladie villeuse gestationnelle, le cancer du col de l'utérus, le cancer de l'endomètre, le sarcome utérin, le cancer de la prostate, le cancer métastatique des os, le cancer métastatique du cerveau, le cancer médiastinal, le cancer rectal, le carcinome rectal, le cancer du vagin, le cancer de la moelle épinière, le schwannome vestibulaire, le cancer du pancréas, le cancer des glandes salivaires, le sarcome de Kaposi, la maladie de Paget, le cancer des amygdales, le carcinome épidermoïde, l'adénocarcinome pulmonaire, le cancer du poumon, le carcinome épidermoïde du poumon, le cancer de la peau, le cancer anal, le rhabdomyosarcome, le cancer du larynx, le cancer de la plèvre, le cancer du sang et le cancer du thymus.

3. Composé pour une utilisation selon la revendication 2, dans lequel le cancer est le cancer du côlon.

4. Composé représenté par la formule 2 ci-dessous, stéréoisomère correspondant, ou sel pharmaceutiquement acceptable correspondant, pour une utilisation pour augmenter l'efficacité d'un agent d'immunothérapie anticancéreuse, dans lequel l'agent d'immunothérapie anticancéreuse est anti-PD1 :

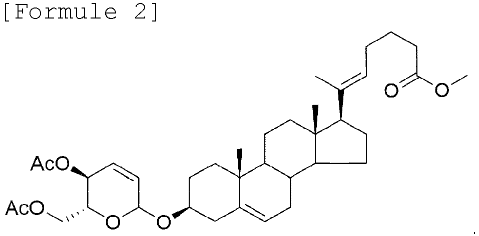

5. Médicament combiné comprenant :
un agent d'immunothérapie anticancéreuse, dans lequel l'agent d'immunothérapie anticancéreuse est anti-PD1 ; et
un composé représenté par la formule 2 ci-dessous, un stéréoisomère correspondant ou un sel pharmaceutiquement acceptable correspondant :

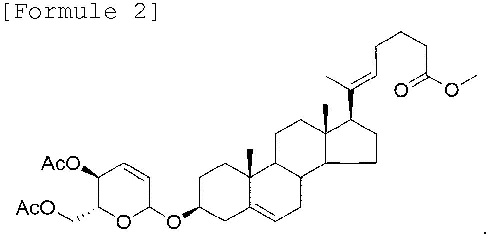

6. Médicament combiné pour une utilisation dans le traitement immunothérapeutique d'un cancer, dans lequel le médicament combiné est défini selon la revendication 5.

7. Médicament combiné pour une utilisation selon la revendication 6, dans lequel le composé, le stéréoisomère correspondant ou le sel pharmaceutiquement acceptable correspondant est administré simultanément ou séquentiellement à l'anti-PD1.

8. Médicament combiné pour une utilisation selon l'une quelconque des revendications 6 ou 7, dans lequel l'utilisation est dans le traitement immunothérapeutique d'au moins un cancer choisi dans le groupe constitué par le pseudomyxome, le cholangiocarcinome intrahépatique, l'hépatoblastome, le cancer du foie, le cancer de la thyroïde, le cancer du côlon, le cancer des testicules, le syndrome myélodysplasique, le glioblastome, le cancer de la bouche, le cancer des lèvres, le mycosis fongoïde, la leucémie myéloïde aiguë, la leucémie aiguë lymphoblastique, le carcinome basocellulaire, le cancer épithélial de l'ovaire, le cancer des cellules germinales de l'ovaire, le cancer du sein masculin, le cancer du cerveau, l'adénome hypophysaire, le myélome multiple, le cancer de la vésicule biliaire, le cancer des voies biliaires, le cancer colorectal, la leucémie myéloïde chronique, la leucémie lymphoïde chronique, le rétinoblastome, le mélanome choroïdien, le cancer de l'ampoule de Vater, le cancer de la vessie, le cancer péritonéal, le cancer de la parathyroïde, le cancer des surrénales, le cancer des fosses nasales, le cancer du poumon non à petites cellules, le cancer de la langue, l'astrocytome, le cancer du poumon à petites cellules, le cancer juvénile du cerveau, le lymphome juvénile, la leucémie juvénile, le cancer de l'intestin grêle, le méningiome, le cancer de l'œsophage, le gliome, le cancer du bassinet, le cancer du rein, le cancer du cœur, le cancer du duodénum, le cancer malin des tissus mous, le cancer malin des os, le lymphome malin, le mésothéliome malin, le mélanome malin, le cancer de l'œil, le cancer de la vulve, le cancer de l'uretère, le cancer de l'urètre, le cancer de site primitif inconnu, le lymphome gastrique, le cancer de l'estomac, le carcinome gastrique, le cancer du stroma gastro-intestinal, le cancer de Wilms, le cancer du sein, le cancer du sein triple négatif, le sarcome, le cancer du pénis, le cancer du pharynx, la maladie villeuse gestationnelle, le cancer du col de l'utérus, le cancer de l'endomètre, le sarcome utérin, le cancer de la prostate, le cancer métastatique des os, le cancer métastatique du cerveau, le cancer médiastinal, le cancer rectal, le carcinome rectal, le cancer du vagin, le cancer de la moelle épinière, le schwannome vestibulaire, le cancer du pancréas, le cancer des glandes salivaires, le sarcome de Kaposi, la maladie de Paget, le cancer des amygdales, le carcinome épidermoïde, l'adénocarcinome pulmonaire, le cancer du poumon, le carcinome épidermoïde du poumon, le cancer de la peau, le cancer anal, le rhabdomyosarcome, le cancer du larynx, le cancer de la plèvre, le cancer du sang et le cancer du thymus.

9. Médicament combiné pour une utilisation selon la revendication 8, dans lequel le cancer est le cancer du côlon.

10. Composé, stéréoisomère correspondant ou sel pharmaceutiquement acceptable correspondant pour une utilisation selon l'une quelconque des revendications 1 à 4, médicament combiné pour une utilisation selon l'une quelconque des revendications 6 à 9, dans lequel le composé, le stéréoisomère correspondant ou le sel pharmaceutiquement acceptable correspondant active au moins un facteur immunitaire choisi dans le groupe constitué par les cellules T auxiliaires, les cellules T cytotoxiques, les cellules tueuses naturelles (cellules NK) et les cytokines.

11. Kit comprenant un agent d'immunothérapie anticancéreuse et un composé représenté par la formule 2 ci-dessous, un stéréoisomère correspondant ou un sel pharmaceutiquement acceptable correspondant,
dans lequel l'agent d'immunothérapie anticancéreuse est anti-PD1 :
